(19) Europäisches Patentamt
European Patent Office
Office européen des brevets

(11) EP 4 413 967 A1

(12) **EUROPEAN PATENT APPLICATION**
published in accordance with Art. 153(4) EPC

(43) Date of publication:
**14.08.2024 Bulletin 2024/33**

(21) Application number: **22878638.0**

(22) Date of filing: **07.10.2022**

(51) International Patent Classification (IPC):
***A61K 6/887*** *(2020.01)* ***A61K 6/60*** *(2020.01)*
***A61K 6/831*** *(2020.01)* ***A61K 6/836*** *(2020.01)*
***A61K 6/84*** *(2020.01)*

(52) Cooperative Patent Classification (CPC):
**A61K 6/60; A61K 6/831; A61K 6/836; A61K 6/84; A61K 6/887**

(86) International application number:
**PCT/JP2022/037733**

(87) International publication number:
**WO 2023/058771 (13.04.2023 Gazette 2023/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.10.2021 JP 2021166529**

(71) Applicant: **Kuraray Noritake Dental Inc.**
**Kurashiki-shi, Okayama 710-0801 (JP)**

(72) Inventors:
- **NOJIRI, Yamato**
  **Tainai-shi, Niigata 959-2653 (JP)**
- **KAWANA, Mariko**
  **Tainai-shi, Niigata 959-2653 (JP)**
- **OKADA, Keishu**
  **Tainai-shi, Niigata 959-2653 (JP)**

(74) Representative: **D Young & Co LLP**
**3 Noble Street**
**London EC2V 7BQ (GB)**

(54) **DIVIDED DENTAL CURING COMPOSITION**

(57) The present invention provides a separate-pack type dental curable composition having favorable chemical polymerization curability, and excellent bond durability to CAD/CAM resins. The present invention relates to a separate-pack type dental curable composition comprising a polymerizable monomer (A) having an acidic group, a polymerizable monomer (B) having no acidic group, an ascorbic acid compound (C), an organic peroxide (D), a transition metal compound (E), and a silane coupling agent (F) represented by the following general formula (f-1). (Description of the symbols in the formula is omitted.)

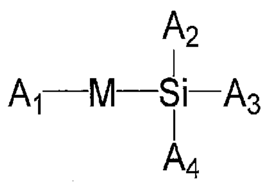

(f-1)

EP 4 413 967 A1

## Description

### TECHNICAL FIELD

The present invention relates to separate-pack type dental curable compositions used in applications such as bonding of dental prostheses, such as crowns, inlays, and bridges, to tooth structures, and construction of abutments in dental treatments. Specifically, the invention relates to a separate-pack type dental curable composition having favorable chemical polymerization curability, and excellent bond durability to CAD/CAM resins.

### BACKGROUND ART

In the restoration of missing teeth caused by damage such as dental caries, prosthetic devices such as inlays, onlays, and crowns are typically created outside the mouth in advance to match the shape of the restored area when the defects are relatively large, and these are subsequently bonded using dental adhesives. Concerning especially aesthetics and metal allergies, there has been a shift from the traditional metal prosthetic devices towards mainstream adoption of prosthetic devices made from CAD/CAM resins or hard resins for crowns through thermal polymerization and/or photopolymerization of composite materials composed of (meth)acrylate polymerizable monomers and inorganic fillers.

Resin-cured materials exhibit a strength comparable to that of teeth upon undergoing polymerization through heat and/or light. Specifically, highly polymerized resin-cured materials by heat, as represented by CAD/CAM resins, display substantial material strength, suitable even for molars enduring considerable occlusal pressure. Typically, dental prostheses created from such resin-cured materials are bonded to abutment teeth using dental resin cements.

However, because of the thermal polymerization involving heat as mentioned above, CAD/CAM resins possess fewer unreacted polymerizable groups on their surfaces compared to photopolymerizable resin materials, reducing their reactivity to dental adhesives. This poses a challenge to adhesive properties in CAD/CAM resins.

Patent Literature 1 proposes a two-paste dental curable composition that exhibits clinically acceptable bond durability to dental adherends, including dental glass-ceramic restorations (particularly, glass-ceramic restorations containing lithium disilicate), without the need to use the traditional primers.

In Patent Literature 2, a polymerization initiator system comprising an organic peroxide selected from hydroperoxides and diperoxides, a transition metal compound, and an ascorbic acid derivative is disclosed as a polymerization initiator system of separate-pack type dental curable compositions having favorable mechanical characteristics and aesthetic properties, along with good adhesive properties.

In Patent Literature 3, a polymerization initiator system comprising an organic peroxide, a thiourea derivative, an ascorbate, and a vanadium compound is proposed as a polymerization initiator system of separate-pack type dental curable compositions that exhibit superior curability in wet conditions, and provide excellent flexural strength in their cured products.

Patent Literature 1: WO2019/004391
Patent Literature 2: WO2016/007453
Patent Literature 3: WO2017/038218

### SUMMARY OF INVENTION

#### Technical Problem

The present inventors examined the compositions described in Patent Literature 1, and found that, while these compositions exhibit high bond durability to glass-ceramic restorations containing lithium disilicate, there remains room for improvement in their bond durability to thermally polymerized CAD/CAM resins involving heat, due to a few unreacted polymerizable groups on their surfaces. It was also found that dental curable compositions containing the polymerization initiator systems disclosed in Patent Literatures 2 and 3 need further improvement in their bond durability to CAD/CAM resins, though these dental curable compositions were found to possess high polymerization curability.

It is accordingly an object of the present invention to provide a separate-pack type dental curable composition having favorable chemical polymerization curability, and excellent bond durability to CAD/CAM resins.

#### Solution to Problem

The present inventors conducted intensive studies, and found that the foregoing issues can be solved with separate-pack type dental curable compositions of specific formulations. This led to the completion of the present

invention after further examinations.

Specifically, the present invention includes the following:

[1] A separate-pack type dental curable composition comprising a polymerizable monomer (A) having an acidic group, a polymerizable monomer (B) having no acidic group, an ascorbic acid compound (C), an organic peroxide (D), a transition metal compound (E), and a silane coupling agent (F) represented by the following general formula (f-1),

[Chem. 1]

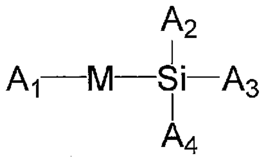

(f－1)

wherein $A_1$ represents a polymerizable functional group selected from the group consisting of a (meth)acryloyloxy group, a vinyl group, and an epoxy group, M represents a divalent aliphatic group having a straight chain with a carbon chain length of 5 or more carbon atoms, or a divalent aromatic group having 6 or more carbon atoms, $A_2$, $A_3$, and $A_4$ each independently represent a hydroxyl group, a C1 to C5 alkyl group, or a C1 to C5 alkoxy group, and at least one of $A_2$, $A_3$,and $A_4$ is a C1 to C5 alkoxy group.

[2] The separate-pack type dental curable composition according to [1], wherein the ascorbic acid compound (C) is at least one compound selected from the group consisting of a salt of ascorbic acid, and an ester of ascorbic acid.

[3] The separate-pack type dental curable composition according to [1] or [2], wherein the transition metal compound (E) is a copper compound or a vanadium compound.

[4] The separate-pack type dental curable composition according to any one of [1] to [3], which is separated into a first agent comprising the polymerizable monomer (A) having an acidic group, and a second agent comprising the silane coupling agent (F).

[5] The separate-pack type dental curable composition according to any one of [1] to [4], wherein M is a divalent aliphatic group having a straight chain with a carbon chain length of 7 or more carbon atoms, or a divalent aromatic group having 7 or more carbon atoms.

[6] The separate-pack type dental curable composition according to any one of [1] to [5], wherein $A_2$, $A_3$, and $A_4$ are each independently a hydroxyl group, a C1 to C3 alkyl group, or a C1 to C3 alkoxy group, and at least one of $A_2$, $A_3$, and $A_4$ is a C1 to C3 alkoxy group.

[7] The separate-pack type dental curable composition according to any one of [1] to [6], wherein $A_2$, $A_3$, and $A_4$ are each independently a hydroxyl group, a methyl group, or a methoxy group, and at least one of $A_2$, $A_3$, and $A_4$ is a methoxy group.

[8] The separate-pack type dental curable composition according to any one of [1] to [7], wherein $A_2$, $A_3$, and $A_4$ are methoxy groups.

[9] The separate-pack type dental curable composition according to any one of [1] to [8], wherein M is an alkylene group having a straight chain with a carbon chain length of 8 or more carbon atoms.

[10] The separate-pack type dental curable composition according to any one of [1] to [9], wherein the silane coupling agent (F) is at least one selected from the group consisting of 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11 -(meth)acryloyloxyundecyltrimethoxysilane, 8- (meth)acryloyloxyoctylmethyldimethoxysilane, 10-(meth)acryloyloxydecylmethyldimethoxysilane, 11-(meth)acryloyloxyundecylmethyldimethoxysilane, and (meth)acryloyloxymethylphenethyltrimethoxysilane.

[11] The separate-pack type dental curable composition according to any one of [1] to [10], wherein the silane coupling agent (F) is at least one selected from the group consisting of 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, and (meth)acryloyloxymethylphenethyltrimethoxysilane.

[12] The separate-pack type dental curable composition according to any one of [1] to [11], wherein the content of the ascorbic acid compound (C) and the content of the silane coupling agent (F) are 1:1 to 1:200 in terms of a mass ratio of ascorbic acid compound (C):silane coupling agent (F).

[13] The separate-pack type dental curable composition according to any one of [1] to [12], which further comprises a filler (G).

[14] The separate-pack type dental curable composition according to any one of [1] to [13], which further comprises a ligand compound.

[15] The separate-pack type dental curable composition according to any one of [1] to [14], which is a dental resin cement.

Advantageous Effects of Invention

According to the present invention, a separate-pack type dental curable composition can be provided that has favorable chemical polymerization curability, and excellent bond durability to CAD/CAM resins.

DESCRIPTION OF EMBODIMENTS

The following describes preferred embodiments of a separate-pack type dental curable composition according to the present invention. It is noted, however, that the present invention is in no way limited by the embodiments described below.

A separate-pack type dental curable composition of the present invention comprises a polymerizable monomer (A) having an acidic group, a polymerizable monomer (B) having no acidic group, an ascorbic acid compound (C), an organic peroxide (D), a transition metal compound (E), and a silane coupling agent (F) represented by the following general formula (f-1),

[Chem. 2]

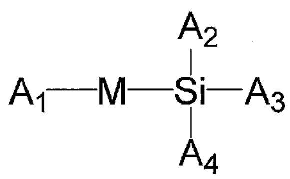

(f − 1)

wherein $A_1$ represents a polymerizable functional group selected from the group consisting of a (meth)acryloyloxy group, a vinyl group, and an epoxy group, M represents a divalent aliphatic group having a straight chain with a carbon chain length of 5 or more carbon atoms, or a divalent aromatic group having 6 or more carbon atoms, $A_2$, $A_3$, and $A_4$ each independently represent a hydroxyl group, a C1 to C5 alkyl group, or a C1 to C5 alkoxy group, and at least one of $A_2$, $A_3$,and $A_4$ is a C1 to C5 alkoxy group.

The mechanism by which a separate-pack type dental curable composition of the present invention accomplishes the effects of the present invention remains unclear. However, the following speculation has been made.

CAD/CAM resins undergo thermal polymerization in their production through polymerization and curing involving heat, resulting in resin surfaces with a few unreacted polymerizable groups. The polymerization initiator system for a composition of the present invention comprising a polymerizable monomer (A) having an acidic group, a polymerizable monomer (B) having no acidic group, an ascorbic acid compound (C), an organic peroxide (D), and a transition metal compound (E) demonstrates high efficiency in initiating polymerization with CAD/CAM resins even under acidic conditions. This enables sufficient improvement of polymerization conversion rate, facilitating efficient crosslinking reactions with the polymerizable groups on the resin surface of CAD/CAM resin, despite a few polymerizable groups. The CAD/CAM resin comprises fillers (such as silica) and polymerizable monomers (such as (meth)acrylic acid esters). The shape of CAD/CAM resin is not particularly limited, and may be, for example, a block shape or disc shape.

In countering the hydrolysis reaction that is responsible for reducing bond durability and occurs in the chemical bonding between the silica in the CAD/CAM resin and the silane coupling agent in the curable composition, the silane coupling agent (F) represented by general formula (f-1) in the present invention exhibits high hydrophobicity attributed to its extended spacer section with a carbon chain length of 5 or more carbon atoms, inhibiting the hydrolysis reaction.

Presumably, these two effects work together synergically without mutual interference, enhancing the bond durability of a separate-pack type dental curable composition of the present invention to CAD/CAM resins. The following describes the components contained in a separate-pack type dental curable composition of the present invention.

The polymerizable monomer (A) having an acidic group of the present invention is an essential component for a dental curable composition of the present invention to exhibit adhesive properties. The polymerizable monomer (A) having an acidic group serves to demineralize tooth structure. The polymerizable monomer (A) having an acidic group is a polymerizable monomer having at least one acidic group such as a phosphoric acid group, a phosphonic acid group, a pyrophosphoric acid group, a thiophosphoric acid group, a carboxylic acid group, and a sulfonic acid group, and at

least one polymerizable group such as an acryloyl group, a methacryloyl group, an acrylamide group, and a methacrylamide group.

In view of adhesive properties, particularly the bond durability to tooth structure and CAD/CAM resins (a polymerized and cured product of a thermally polymerizable resin material containing a thermal polymerization initiator), the polymerizable monomer (A) having an acidic group is preferably a monofunctional polymerizable monomer having any one of an acryloyl group, a methacryloyl group, an acrylamide group, and a methacrylamide group as a polymerizable group. Specific examples of the polymerizable monomer (A) having an acidic group are as follows.

Examples of the polymerizable monomer having a phosphoric acid group include:

monofunctional (meth)acrylate compounds having a phosphoric acid group, such as 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, 2-(meth)acryloyloxyethylphenyl hydrogen phosphate, 2-(meth)acryloyloxyethyl-2-bromoethyl hydrogen phosphate, 2-(meth)acryloyloxyethyl-(4-methoxyphenyl)hydrogen phosphate, 2-(meth)acryloyloxypropyl-(4-methoxyphenyl)hydrogen phosphate, and acid chlorides, alkali metal salts, ammonium salts, and amine salts of these; and

bifunctional (meth)acrylate compounds having a phosphoric acid group, such as bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, and acid chlorides, alkali metal salts, ammonium salts, and amine salts of these.

Examples of the polymerizable monomer having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 1 0-(meth)acryloyloxydecyl-3-phosphonopropionate, 6-(meth)acryloyloxyhexylphosphonoacetate, 10-(meth)acryloyloxydecylphosphonoacetate, and acid chlorides, alkali metal salts, ammonium salts, and amine salts of these.

Examples of the polymerizable monomer having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, ammonium salts, and amine salts of these.

Examples of the polymerizable monomer having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyeicosyl dihydrogen thiophosphate, and acid chlorides, alkali metal salts, and ammonium salts of these.

Examples of the polymerizable monomer having a carboxylic acid group include:

(meth)acrylic acid, 4-[2-[(meth)acryloyloxy]ethoxycarbonyl]phthalic acid, 4-(meth)acryloyloxyethyl trimellitic acid, 4-(meth)acryloyloxybutyloxycarbonyl phthalic acid, 4-(meth)acryloyloxyhexyloxycarbonyl phthalic acid, 4-(meth)acryloyloxyoctyloxycarbonyl phthalic acid, 4-(meth)acryloyloxydecyloxycarbonyl phthalic acid, and acid anhydrides of these; and

5-(meth)acryloylaminopentylcarboxylic acid, 6-(meth)acryloyloxyhexane-1,1-dicarboxylic acid, 8-(meth)acryloyloxyoctane-1,1-dicarboxylic acid, 10-(meth)acryloyloxydecane-1,1 -dicarboxylic acid, 11-(meth)acryloyloxyundecane-1,1-dicarboxylic acid, and acid chlorides, alkali metal salts, ammonium salts, and amine salts of these.

Examples of the polymerizable monomer having a sulfonic acid group include 2-(meth)acrylamide-2-methylpropanesulfonic acid, 2-sulfoethyl (meth)acrylate, and acid chlorides, alkali metal salts, ammonium salts, and amine salts of these.

In view of providing even superior adhesive properties to tooth structure, and even superior adhesive properties, particularly the bond durability to CAD/CAM resins, preferred among these polymerizable monomers (A) having an acidic group are polymerizable monomers having a phosphoric acid group, polymerizable monomers having a pyrophosphoric

acid group, and polymerizable monomers having a carboxylic acid group, more preferably polymerizable monomers having a phosphoric acid group, polymerizable monomers having a carboxylic acid group.

Even more preferred are (meth)acrylate monofunctional polymerizable monomers having a phosphoric acid group, or (meth)acrylate polymerizable monomers having a carboxylic acid group with a C6 to C20 alkyl group or C6 to C20 alkylene group as the backbone within the molecule, more preferably (meth)acrylate monofunctional polymerizable monomers having a phosphoric acid group with a C8 to C12 alkylene group as the backbone within the molecule.

Particularly preferred are 10-methacryloyloxydecyl dihydrogen phosphate, 4-(meth)acryloyloxyethyl trimellitic acid, and 4-(meth)acryloyloxyethyl trimellitic acid anhydride, most preferably 10-methacryloyloxydecyl dihydrogen phosphate.

The polymerizable monomer (A) having an acidic group may be incorporated alone, or two or more thereof may be incorporated in combination.

The content of polymerizable monomer (A) having an acidic group is not particularly limited, as long as the present invention can exhibit its effects. However, in view of even superior adhesive properties, particularly the bond durability to CAD/CAM resins, the content of polymerizable monomer (A) having an acidic group ranges preferably from 1 to 50 parts by mass, more preferably from 2 to 25 parts by mass, even more preferably from 2 to 10 parts by mass in total 100 parts by mass of polymerizable monomer components in a separate-pack type dental curable composition of the present invention.

In this specification, the phrase "total 100 parts by mass of polymerizable monomer components in a dental curable composition" means the content of when the total amount of the polymerizable monomers contained in the first and second agents is converted to 100 parts by mass.

In this specification, "total amount of dental curable composition" means the total amount of the components contained in the first and second agents.

A separate-pack type dental curable composition of the present invention comprises a polymerizable monomer (B) having no acidic group. The polymerizable monomer (B) having no acidic group is a polymerizable monomer that undergoes polymerization as a result of a radical polymerization reaction driven by a polymerization initiator system. The polymerizable monomer (B) having no acidic group may be used alone, or two or more thereof may be used in combination.

Preferred examples of the polymerizable monomer (B) having no acidic group include the hydrophilic polymerizable monomer (B-1) and hydrophobic polymerizable monomer (B-2) below.

Hydrophilic polymerizable monomer (B-1) means a polymerizable monomer having a solubility of 10 mass% or more in water at 25°C. Preferably, the hydrophilic polymerizable monomer (B-1) is one having a solubility of 30 mass% or more in water at 25°C, more preferably one that can dissolve in water in any proportion at 25°C.

The hydrophilic polymerizable monomer (B-1) promotes penetration of the components of the dental curable composition into tooth structure. The hydrophilic polymerizable monomer (B-1) itself also penetrates into tooth structure, and adheres to the organic component (collagen) in the tooth structure.

Examples of the hydrophilic polymerizable monomer (B-1) include monofunctional (meth)acrylic acid ester polymerizable monomers such as 2-hydroxyethyl acrylate, 2-hydroxyethyl methacrylate (hereinafter, also referred to by the abbreviation "HEMA"), 3-hydroxypropyl (meth)acrylate, 2-hydroxypropyl (meth)acrylate, 1,3-dihydroxypropyl (meth)acrylate, 2,3-dihydroxypropyl (meth)acrylate, and 2-((meth)acryloyloxy)ethyltrimethylammonium chloride; and bifunctional (meth)acrylic acid ester polymerizable monomers such as polyethylene glycol di(meth)acrylate (with an average of 9 or more moles of oxyethylene group added). 2-Hydroxyethyl (meth)acrylate is preferred in view of exhibiting even superior adhesive properties to tooth structure, and even superior adhesive properties, particularly the bond durability to CAD/CAM resins.

In this specification, "(meth)acryl" means acryl and methacryl, and the same applies to similar expressions, such as "(meth)acryloyl" and "(meth)acrylate".

Hydrophobic polymerizable monomer (B-2) means a crosslinkable polymerizable monomer having a solubility of less than 10 mass% in water at 25°C.

Examples of the hydrophobic polymerizable monomer (B-2) include aromatic monofunctional polymerizable monomers and bifunctional polymerizable monomers, aliphatic monofunctional polymerizable monomers and bifunctional polymerizable monomers, and tri- and higher-functional polymerizable monomers. The hydrophobic polymerizable monomer (B-2) improves properties such as the mechanical strength of cured products of the dental curable composition, and ease of handling.

Examples of the aromatic monofunctional polymerizable monomers include benzyl (meth)acrylate, p-cumyl-phenoxyethylene glycol (meth)acrylate, and 2-phenoxybenzyl (meth)acrylate. Preferred among these are benzyl methacrylate, and p-cumyl-phenoxyethylene glycol methacrylate.

Examples of the aromatic bifunctional polymerizable monomers include aromatic di(meth)acrylates. Specific examples of the aromatic bifunctional polymerizable monomers include 2,2-bis((meth)acryloyloxyphenyl)propane, 2,2-bis[4-(3-acryloyloxy-2-hydroxypropoxy)phenyl]propane, 2,2-b is[4-(2-hydroxy-3-m ethacryloyloxypropoxy) phenyl] propane (hereinafter, also referred to by the abbreviation "Bis-GMA"), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromellitate. Preferred among these are 2,2-bis[4-(3-methacryloyloxy-2-hydroxypropoxy)phenyl]propane, and 2,2-bis(4-methacryloyloxypolyethoxyphenyl)propane (with an average of 2.6 moles of ethoxy group added; hereinafter, also referred to by the abbreviation "D-2.6E").

Examples of the aliphatic monofunctional polymerizable monomers include methyl (meth)acrylate, ethyl (meth)acrylate, and butyl (meth)acrylate. Other examples include isobornyl (meth)acrylate, stearyl (meth)acrylate, dicyclopentanyl (meth)acrylate, butoxydiethylene glycol (meth)acrylate, and methoxypolyethylene glycol (meth)acrylate. Preferred among these is isobornyl methacrylate.

Examples of the aliphatic bifunctional polymerizable monomers include:

bifunctional (meth)acrylic acid ester polymerizable monomers such as erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol diacrylate, triethylene glycol dimethacrylate (hereinafter, also referred to by the abbreviation "TEGDMA"), propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylenebis(2-carbamoyloxyethyl)dimethacrylate, and 1,2-bis(3-methacryloyloxy-2-hydroxypropyloxy)ethane; and

(meth)acrylamide polymerizable monomers such as N-methacryloyloxyethylacrylamide, N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.

Preferred among these are glycerol dimethacrylate, triethylene glycol di(meth)acrylate, neopentyl glycol dimethacrylate, 2,2,4-trimethylhexamethylenebis(2-carbamoyloxyethyl)dimethacrylate, and 1,2-bis(3-methacryloyloxy-2-hydroxypropyloxy)ethane.

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol]tetramethacryl ate, and 1,7-diacryloyloxy-2,2,6,6-tetraacryloyloxymethyl-4-oxaheptane.

In view of the bond strength and polymerization curability of a dental curable composition of the present invention, preferred among these polymerizable monomers (B) having no acidic group are HEMA, Bis-GMA, D-2.6E, and TEGDMA.

The polymerizable monomer (B) having no acidic group (hydrophilic polymerizable monomer (B-1), hydrophobic polymerizable monomer (B-2)) may be incorporated alone, or two or more thereof may be incorporated in combination.

The content of polymerizable monomer (B) containing no acidic group is not particularly limited, as long as the present invention can exhibit its effects. However, in view of providing a composition having high penetrability into the tooth structure and superior adhesive properties, as well as providing a cured product having a sufficient mechanical strength, the content of polymerizable monomer (B) containing no acidic group ranges preferably from 50 to 99 parts by mass, more preferably from 60 to 98 parts by mass, even more preferably from 70 to 95 parts by mass in total 100 parts by mass of polymerizable monomer components in a separate-pack type dental curable composition of the present invention.

The following describes the polymerization initiator system.

A separate-pack type dental curable composition of the present invention comprises an ascorbic acid compound (C), an organic peroxide (D), and a transition metal compound (E) as its polymerization initiator system. A separate-pack type dental curable composition of the present invention exhibits favorable chemical polymerization curability, and excellent bond durability to CAD/CAM resins by using such a polymerization initiator system with other components. With the polymerization initiator system used with other components, it is also possible to set an adequate working time range for the separate-pack type dental curable composition in use.

Examples of the ascorbic acid compound (C) include salts, esters, and ethers of ascorbic acid. In view of enhancing the effectiveness of the present invention, preferred are salts and esters of ascorbic acid. The presence of the polymerization initiator system containing an ascorbic acid compound (C) provides high efficiency in initiating polymerization even under acidic conditions. This enables sufficient improvement of polymerization conversion rate, providing favorable chemical polymerization curability, and facilitating efficient crosslinking reactions between the polymerizable groups and the components contained in a separate-pack type dental curable composition of the present invention, despite a small number of polymerizable groups on the surface of CAD/CAM resin. The combined effect of this action, alongside the inhibition of hydrolysis reaction enabled by the use of silane coupling agent (F), works synergically without mutual interference, enhancing the adhesive properties, particularly the bond durability to CAD/CAM resins.

Examples of salts of ascorbic acid include sodium L-ascorbate, calcium L-ascorbate, potassium ascorbate, and stereoisomers of these (for example, sodium isoascorbate). Preferred among these is sodium L-ascorbate.

Examples of esters of ascorbic acid include those formed by a reaction of carboxylic acid with one or more of the hydroxyl groups of ascorbic acid.

Preferred examples of carboxylic acids include fatty acids such as C6 to C30 saturated fatty acids or unsaturated fatty acids, for example, caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, $\alpha$-linolenic acid, arachidonic acid, eicosapentaenoic acid, erucic acid, and docosahexaenoic acid.

The fatty acids have preferably 10 to 28 carbon atoms, more preferably 12 to 26 carbon atoms, even more preferably 14 to 24 carbon atoms. Particularly preferred among these are esters of stearic acid and ascorbic acid, and esters of palmitic acid and ascorbic acid (ascorbyl palmitate).

Examples of ethers of ascorbic acid include ethyl ascorbic ether, and cetyl ascorbic ether.

The ascorbic acid compound (C) may be incorporated alone, or two or more thereof may be incorporated in combination. In view of curability, the mechanical strength of the cured product, and adhesive properties, particularly the bond durability to tooth structure and CAD/CAM resins, the content of ascorbic acid compound (C) ranges preferably from 0.01 to 8 parts by mass, more preferably from 0.1 to 5 parts by mass, even more preferably from 0.5 to 2 parts by mass, particularly preferably from 0.8 to 2 parts by mass with respect to total 100 parts by mass of polymerizable monomer components in a separate-pack type dental curable composition of the present invention. In view of the particular enhancement of these effects, the range of 1.2 to 2 parts by mass is most preferred.

In view of curability, the mechanical strength of the cured product, and adhesive properties, particularly the bond durability to tooth structure and CAD/CAM resins, the content of ascorbic acid compound (C) is preferably 0.001 to 7.0 mass%, more preferably 0.01 to 5.0 mass%, even more preferably 0.1 to 2.0 mass%, particularly preferably 0.2 to 1.5 mass% in a total amount of a separate-pack type dental curable composition of the present invention. In view of the particular enhancement of these effects, the range of 0.4 to 1.5 mass% is most preferred.

The ascorbic acid compound (C) may be dissolved in the composition, or may be dispersed therein in powder form.

When the ascorbic acid compound (C) is dispersed in powder form, the average particle diameter of the powder is preferably 20 $\mu$m or less, more preferably 10 $\mu$m or less, even more preferably 5 $\mu$m or less, because workability or curability tends to decrease when the average particle diameter is excessively large.

The average particle diameter of a powder of ascorbic acid compound (C) can be calculated as a volume average particle diameter after an image analysis with image-analyzing particle size distribution measurement software (Mac-View, manufactured by Mountech Co., Ltd.) based on an electron micrograph of at least 100 particles.

The particle shape of when the ascorbic acid compound (C) is dispersed in powder form is not particularly limited, and the particles may have various shapes, for example, such as spherical, needle-like, plate-like, and crushed shapes. The ascorbic acid compound (C) can be prepared by known methods such as pulverization, freeze drying, and reprecipitation. In view of the average particle diameter of the resultant powder, preferred are pulverization and freeze drying.

Examples of the organic peroxide (D) include diacyl peroxides, peroxyesters, dialkyl peroxides, peroxyketals, ketone peroxides, and hydroperoxides.

Specific examples of the diacyl peroxides include benzoyl peroxide, 2,4-dichlorobenzoyl peroxide, and m-toluoyl peroxide.

Specific examples of the peroxyesters include t-butyl peroxybenzoate, bis(t-butylperoxy)isophthalate, 2,5-dimethyl-2,5-bis(benzoylperoxy)hexane, t-butyl peroxy-2-ethylhexanoate, and t-butyl peroxyisopropyl carbonate.

Specific examples of the dialkyl peroxides include dicumyl peroxide, di-t-butyl peroxide, and lauroyl peroxide.

Specific examples of the peroxyketals include 1,1-bis(t-butylperoxy)3,3,5-trimethylcyclohexane, 1,1-bis(t-butylperoxy)cyclohexane, and 1,1-bis(t-hexylperoxy)cyclohexane.

Specific examples of the ketone peroxides include methyl ethyl ketone peroxide, cyclohexanone peroxide, and methyl acetoacetate peroxide.

Specific examples of the hydroperoxides include t-butyl hydroperoxide, cumene hydroperoxide, p-diisopropylbenzene hydroperoxide, and 1,1,3,3-tetramethylbutyl hydroperoxide.

Particularly preferred as organic peroxide (D) are hydroperoxides and peroxyesters. Preferred for use as hydroperoxides are t-butyl hydroperoxide, cumene hydroperoxide, and 1,1,3,3-tetramethylbutyl hydroperoxide. Preferred

for use as peroxyesters is t-butyl peroxybenzoate.

The organic peroxide (D), when used with other components, provides superior chemical polymerization curability, and excellent adhesive properties, particularly the bond durability to CAD/CAM resins. The organic peroxide (D) may be incorporated alone, or two or more thereof may be incorporated in combination.

In view of chemical polymerization curability, and adhesive properties, particularly the bond durability to CAD/CAM resins, the content of organic peroxide (D) ranges preferably from 0.01 to 10 parts by mass, more preferably from 0.1 to 5 parts by mass, even more preferably from 0.5 to 3 parts by mass with respect to total 100 parts by mass of polymerizable monomer components in a separate-pack type dental curable composition of the present invention. The content of organic peroxide (D) within the foregoing ranges enables adjustment to the desired working time.

Preferred for use as the transition metal compound (E) are copper compounds and vanadium compounds.

Examples of the copper compounds include copper(II) carboxylates, copper(II) β-diketones, copper(II) β-ketoesters, copper alkoxides, copper dithiocarbamates, and salts of copper and inorganic acids.

Examples of the copper(II) carboxylates include copper(II) citrate, copper(II) acetate, copper(II) phthalate, copper(II) tartarate, copper(II) oleate, copper(II)octylate, copper(II) octenate, copper(II) naphthenate, copper(II) methacrylate, and copper(II) 4-cyclohexylbutyrate.

Examples of the copper(II) β-diketones include copper(II) acetylacetonate, copper(II) trifluoroacetylacetonate, copper(II) hexafluoroacetylacetonate, copper(II) 2,2,6,6-tetramethyl-3,5-heptanedionate, and copper(II) benzoylacetonate.

Examples of the copper(II) β-ketoesters include copper(II) ethylacetoacetate.

Examples of the copper alkoxides include copper(II) methoxide, copper(II) ethoxide, copper(II) isopropoxide, copper(II) 2-(2-butoxyethoxy)ethoxide, and copper(II) 2-(2-methoxyethoxy)ethoxide.

Examples of the copper dithiocarbamates include copper(II) dimethyldithiocarbamate.

Examples of the salts of copper and inorganic acids include copper(II) nitrate, copper(II) bromide, and copper(II) chloride.

These may be used alone, or two or more thereof may be used in appropriate combinations. In view of solubility and reactivity to the polymerizable monomers, preferred are copper(II) carboxylates, copper(II) β-diketones, and copper(II) β-ketoesters, more preferably copper(II) acetate, and copper(II) acetylacetonate.

Preferred as the vanadium compounds are vanadium compounds with a valency of IV and/or V.

Examples of the vanadium compounds with a valency of IV and/or V include vanadium(IV) oxide, vanadyl(IV) acetylacetonate, vanadium(IV) oxide stearate, oxalic acid oxovanadium(IV), vanadyl(IV) sulfate, vanadium naphthenate, vanadium benzoylacetonate, bis(maltolato)oxovanadium(IV), oxobis(1-phenyl-1,3-butanedionate)vanadium(IV), vanadium(V) oxide, vanadium(V) oxytriisopropoxide, sodium metavanadate, and ammonium metavanadate(V).

In view of considerations such as adhesive properties, preferred are vanadium(IV) oxide, vanadyl(IV) sulfate, vanadyl(IV) acetylacetonate, and bis(maltolato)oxovanadium(IV), more preferably vanadyl(IV) acetylacetonate and bis(maltolato)oxovanadium(IV). The vanadium compounds may be used alone, or two or more thereof may be used in combination.

The transition metal compound (E), when used with other components, provides superior chemical polymerization curability, and excellent adhesive properties, particularly the bond durability to CAD/CAM resins.

In view of chemical polymerization curability, and adhesive properties, particularly the bond durability to CAD/CAM resins, the content of transition metal compound (E) ranges preferably from 0.0001 to 1 part by mass, more preferably from 0.0005 to 0.5 parts by mass, even more preferably from 0.001 to 0.2 parts by mass with respect to total 100 parts by mass of polymerizable monomer components in a separate-pack type dental curable composition of the present invention. The content of transition metal compound (E) within the foregoing ranges enables adjustment to the desired working time.

In general formula (f-1) representing the silane coupling agent (F), $A_1$ represents a polymerizable functional group selected from the group consisting of a (meth)acryloyloxy group, a vinyl group, and an epoxy group, M represents a divalent aliphatic group having a straight chain with a carbon chain length of 5 or more carbon atoms, or a divalent aromatic group having 6 or more carbon atoms, $A_2$, $A_3$, and $A_4$ each independently represent a hydroxyl group, a C1 to C5 alkyl group, or a C1 to C5 alkoxy group, and at least one of $A_2$, $A_3$,and $A_4$ is a C1 to C5 alkoxy group.

In view of polymerizability such as in the copolymerization of the silane coupling agent (F) with the polymerizable monomers (polymerizable monomer (A) having an acidic group, and polymerizable monomer (B) having no acidic group), $A_1$ is preferably a (meth)acryloyloxy group, more preferably a methacryloyloxy group.

The divalent aliphatic group represented by M may have a branched chain in addition to a straight chain, provided that it is a divalent aliphatic group having a straight chain with a carbon chain length of 5 or more carbon atoms. The presence of a straight chain with a carbon chain length of 5 or more carbon atoms is thought to aid in aligning molecules upon bonding to CAD/CAM resin, thereby improving the hydrophobicity of the bonding interface.

In view of more greatly enhancing the hydrophobicity of the bonding interface, the aliphatic group is preferably a linear aliphatic group with a carbon chain length of 5 or more carbon atoms. The aliphatic group may consist of only

carbon atoms and hydrogen atoms, or may contain a heteroatom such as an oxygen atom, a nitrogen atom, or a sulfur atom.

In view of the ability to more greatly enhance the hydrophobicity of the bonding interface, the straight chain in the aliphatic group represented by M has a carbon chain length of preferably 6 or more carbon atoms, more preferably 7 or more carbon atoms, even more preferably 8 or more carbon atoms.

The straight chain in the aliphatic group represented by M has a carbon chain length of preferably 20 or fewer carbon atoms, more preferably 15 or fewer carbon atoms, even more preferably 12 or fewer carbon atoms. Examples of the aliphatic group include alkylene groups, alkenylene groups, and alkynylene groups. Preferred are alkylene groups.

Examples of the alkylene groups include a pentamethylene group, a hexamethylene group, a heptamethylene group, an octamethylene group, a nonamethylene group, a decamethylene group, an undecamethylene group, and a dodecamethylene group.

A certain preferred embodiment is, for example, a separate-pack type dental curable composition comprising a silane coupling agent (F) in which M in general formula (f-1) is an alkylene group having a straight chain with a carbon chain length of 8 or more carbon atoms.

The aliphatic group or aromatic group represented by M may contain any divalent group. Examples of such divalent groups include bonds other than carbon-carbon bonds, such as an ether group, an ester group, an amide group, a sulfonyl group, a urethane group, and a thioether group. The alkylene group has preferably 1 to 15 carbon atoms, more preferably 1 to 10 carbon atoms, even more preferably 1 to 8 carbon atoms, particularly preferably 1 to 6 carbon atoms.

The divalent aliphatic group and divalent aromatic group represented by M may have a substituent. Examples of the substituent include halogen atoms; functional groups with no carbon atoms, such as a hydroxyl group, an oxo group, an amino group, a cyano group, and a nitro group; C1 to C6 alkyl groups; C1 o C6 alkoxy groups; and C2 to C6 alkenyl groups. The number of substituents is preferably 1 to 10, more preferably 1 to 8, even more preferably 1 to 4.

The divalent aromatic group represented by M may be a homocyclic ring group consisting of only carbon atoms, or may be a heterocyclic ring group. Examples of the heteroatoms contained in the heterocyclic ring group include nitrogen atoms, sulfur atoms, and oxygen atoms. The heterocyclic ring group contains preferably 1 to 3 heteroatoms.

The aromatic group has 6 or more carbon atoms, preferably 7 or more carbon atoms, more preferably 8 or more carbon atoms, even more preferably 9 or more carbon atoms. The aromatic group has preferably 35 or fewer carbon atoms, more preferably 20 or fewer carbon atoms, even more preferably 16 or fewer carbon atoms.

Examples of the divalent aromatic group include alkylene group-containing arylene groups, and arylene groups.

Examples of the arylene groups include phenylene groups; and polycyclic aromatic groups such as a naphthylene group, an anthracenylene group, a phenanthrylene group, a biphenylene group, and a fluorenylene group.

Examples of the alkylene group in the alkylene group-containing arylene groups include a methylene group, an ethylene group, an n-propylene group, an isopropylene group, a butylene group, a pentylene group, a hexylene group, a heptylene group, an octylene group, a nonylene group, a decylene group, and a dodecylene group. The aromatic group represented by M may be bonded to the alkylene groups at both of the two bonding sites

Examples of the alkylene group-containing arylene groups include an ethylenephenylene group, a diethylenephenylene group, a triethylenephenylene group, a propylenephenylene group, and a butylenephenylene group. When the aromatic group is an alkylene group-containing arylene group having a substituent, the substituent may be present on the aromatic ring or on the alkylene group. The aromatic group represented by M is preferably an alkylene group-containing arylene group. In view of more greatly enhancing the hydrophobicity of the bonding interface, the bonding sites of the divalent aromatic group are located preferably at the para position of the aromatic ring.

The alkyl groups represented by $A_2$, $A_3$, and $A_4$ have preferably 1 to 3 carbon atoms, more preferably 1 carbon atom.

The alkoxy groups represented by $A_2$, $A_3$, and $A_4$ have preferably 1 to 3 carbon atoms. In view of bond durability to CAD/CAM resins, the carbon count is more preferably 1.

Examples of the alkoxy groups represented by $A_2$, $A_3$, and $A_4$ include a methoxy group, an ethoxy group, an n-propoxy group, an isopropoxy group, an n-butoxy group, a sec-butoxy group, and a tert-butoxy group. Examples of the alkyl groups include a methyl group, an ethyl group, an n-propyl group, an isopropyl group, an n-butyl group, and an isobutyl group. In view of bond durability to CAD/CAM resins, the alkoxy groups represented by $A_2$, $A_3$, and $A_4$ are more preferably methoxy groups.

Favorable bond durability to CAD/CAM resins can be achieved by incorporating the silane coupling agent (F) within the dental curable composition besides the surface treatment agent used for the filler, even in the absence of primers.

This is because the silane coupling agent (F), by containing a compound having an extended spacer section with a carbon chain length of 5 or more carbon atoms, can sufficiently hydrophobize the bonding interface due to its high hydrophobicity, and inhibit the hydrolysis reaction that is responsible for reducing bond durability and occurs in the chemical bonding between the silica in the CAD/CAM resin and the silane coupling agent in the dental curable composition.

Besides the effect to inhibit hydrolysis reaction, the polymerization initiator system provides high efficiency in initiating polymerization even under acidic conditions, enabling sufficient improvement of polymerization conversion rate. This facilitates efficient crosslinking reactions between the polymerizable groups and the components contained in a separate-pack type dental curable composition of the present invention, despite a small number of polymerizable groups on the surface of CAD/CAM resin. These two effects work together synergically without mutual interference, providing excellent bond durability to CAD/CAM resins.

The silane coupling agent (F) may be used alone, or two or more thereof may be used in combination.

The silane coupling agent (F) may be any known silane coupling agents satisfying general formula (f-1).

Specific examples of silane coupling agent (F) include vinyl group-containing silane coupling agents such as vinylhexyltrimethoxysilane, vinylheptyltrimethoxysilane, and vinyloctyltrimethoxysilane; and (meth)acryloyloxy group-containing silane coupling agents such as 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, 8-(meth)acryloyloxyoctylmethyldimethoxysilane, 10-(meth)acryloyloxydecylmethyldimethoxysilane, 11-(meth)acryloyloxyundecylmethyldimethoxysilane, and (meth)acryloyloxymethylphenethyltrimethoxysilane.

Particularly, in view of bond durability to CAD/CAM resins and ease of handling, preferred for use among these silane coupling agents (F) are 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, and (meth)acryloyloxymethylphenethyltrimethoxysilane.

In view of providing superior bond strength, the content of silane coupling agent (F) is preferably 0.1 to 10.0 mass% in a total amount of a separate-pack type dental curable composition of the present invention. In view of bond durability to CAD/CAM resins, the content of silane coupling agent (F) is more preferably 0.5 to 9.0 mass%, even more preferably 1.0 to 8.0 mass%, particularly preferably 1.2 to 7.0 mass%.

In view of curability, the mechanical strength of the cured product, and adhesive properties, particularly the bond durability to tooth structure and CAD/CAM resins, it is preferable in certain preferred embodiments that the content of ascorbic acid compound (C) and the content of silane coupling agent (F) be preferably 1:1 to 1:200, more preferably 1:1.5 to 1:150, even more preferably 1:2 to 1:80, particularly preferably 1:3 to 1:70 in terms of a mass ratio of ascorbic acid compound (C):silane coupling agent (F) in a separate-pack type dental curable composition of the present invention. In view of the particular enhancement of these effects, the ratio of 1:4 to 1:50 is most preferred.

A certain preferred embodiment is, for example, a separate-pack type dental curable composition that is separated into a first agent comprising the polymerizable monomer (A) having an acidic group, and a second agent comprising the silane coupling agent (F). Preferably, the polymerizable monomer (A) having an acidic group, and the silane coupling agent (F) are separately incorporated to inhibit the hydrolysis of silane coupling agent (F), and improve the storage stability during the storage of the separate-pack type dental curable composition.

In a separate-pack type dental curable composition of the present invention, at least one of the first and second agents may comprise a filler (G) to provide sufficient workability in the composition, and achieve sufficient X-ray opacity and mechanical strength in the cured product.

The filler (G) may be any type of filler, provided that it does not hinder the effectiveness of the present invention. Examples include inorganic fillers, organic fillers, and composite fillers of inorganic fillers and organic fillers. The filler (G) may be incorporated alone, or two or more thereof may be incorporated in combination. The filler (G) has an average particle diameter of preferably 0.001 to 10 μm, more preferably 0.001 to 5 μm.

Examples of the inorganic fillers include silica; silica-based minerals such as kaolin, clay, *ummo,* and mica; and various types of ceramic and glass containing base material silica and other components such as $Al_2O_3$, $B_2O_3$, $TiO_2$, $ZrO_2$, BaO, $La_2O_3$, SrO, ZnO, CaO, $P_2O_5$, $Li_2O$, and $Na_2O$. Examples of such glass include lithium borosilicate glass, borosilicate glass, bioglass, lanthanum glass, barium glass, strontium glass, soda glass, zinc glass, and fluoroaluminosilicate glass. Also preferred for use as inorganic fillers are crystal quartz, hydroxyapatite, alumina, titanium oxide, yttrium oxide, zirconia, barium sulfate, aluminum hydroxide, sodium fluoride, potassium fluoride, sodium monofluorophosphate, lithium fluoride, and ytterbium fluoride.

In view of adhesive properties and ease of handling, preferred for use is particulate silica having an average particle diameter of 0.001 to 0.1 μm.

A certain preferred embodiment is, for example, a separate-pack type dental curable composition that further comprises a filler (G), and the filler (G) comprises an inorganic filler having an average particle diameter of 0.001 μm or more and 0.1 μm or less, and an inorganic filler having an average particle diameter of more than 0.1 μm and 10 μm or less.

The inorganic fillers may be commercially available products. Examples of such commercially available products include Aerosil® OX50, Aerosil® 50, Aerosil® 200, Aerosil® 380, Aerosil® R972, Aerosil® 130, and AEROXIDE® Alu C (all manufactured by Nippon Aerosil Co., Ltd. under these trade names).

In the present invention, the average particle diameter of inorganic filler means the average particle diameter

before surface treatment when the inorganic filler is surface treated as will be described later.

Examples of the organic fillers include polymethyl methacrylate, polyethyl methacrylate, polymers of polyfunctional methacrylate, polyamides, polystyrene, polyvinyl chloride, chloroprene rubber, nitrile rubber, and styrene-butadiene rubber.

Examples of the composite fillers of inorganic fillers and organic fillers include those comprising inorganic fillers dispersed in organic fillers, and inorganic/organic composite fillers comprising various types of polymers coating the inorganic filler.

The filler (G) may be used after a surface treatment with a known surface treatment agent such as a silane coupling agent, in order to improve curability, mechanical strength, and ease of handling. Examples of such surface treatment agents include vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, and γ-aminopropyltriethoxysilane.

The average particle diameter (average primary particle diameter) can be determined by a laser diffraction scattering method or by electron microscopy of particles. Specifically, a laser diffraction scattering method is more convenient for the measurement of particles that are 0.1 μm or larger, whereas electron microscopy is a more convenient method of particle diameter measurement for ultrafine particles of less than 0.1 μm. Here, a value of 0.1 μm is the reference value, measured by a laser diffraction scattering method.

As an example of a laser diffraction scattering method, the particle size may be measured by volume using a laser diffraction particle size distribution analyzer (SALD-2300, manufactured by Shimadzu Corporation) with a 0.2% sodium hexametaphosphate aqueous solution used as dispersion medium.

A scanning electron microscope (e.g., SU3800 or S-4000 manufactured by Hitachi High-Technologies Corporation) may be used for electron microscopy. In electron microscopy, the particle size can be measured by taking an electron micrograph of particles, and the size of particles (at least 200 particles) observed in a unit field of the captured image may be measured using image-analyzing particle size distribution measurement software (Mac-View, manufactured by Mountech Co., Ltd.). Here, the particle diameter is determined as an arithmetic mean value of the maximum and minimum lengths of particles, and the average primary particle diameter is calculated from the number of particles and the particle diameter.

The content of filler (G) is not particularly limited, as long as the present invention can exhibit its effects. However, the content of filler (G) ranges preferably from 20 to 500 parts by mass, more preferably from 50 to 300 parts by mass, even more preferably from 100 to 250 parts by mass with respect to total 100 parts by mass of polymerizable monomer components in a separate-pack type dental curable composition of the present invention. With the content of filler (G) confined within these ranges, the cured product can have sufficient X-ray opacity or mechanical strength, and the paste can exhibit sufficient workability.

The content of filler (G) is not particularly limited, as long as the present invention can exhibit its effects. However, the content of filler (G) is preferably 50 to 97 mass%, more preferably 55 to 95 mass%, even more preferably 60 to 90 mass% in a total amount of a separate-pack type dental curable composition of the present invention.

A separate-pack type dental curable composition of the present invention may comprise a ligand compound. In certain preferred embodiments, the ligand compound is at least one compound selected from the group consisting of a ligand containing a phosphorus atom, and a ligand containing a nitrogen atom. The ligand containing a phosphorus atom contains a phosphorus atom as a coordinating atom. The ligand containing a nitrogen atom contains a nitrogen atom as a coordinating atom.

Another certain embodiment is, for example, a separate-pack type dental curable composition that further comprises a ligand compound. A separate-pack type dental curable composition comprising a ligand compound also exhibits favorable chemical polymerization curability, and excellent bond durability to CAD/CAM resins.

A certain preferred embodiment is, for example, a separate-pack type dental curable composition in which the ligand compound comprises a ligand containing a nitrogen atom.

Another preferred embodiment is, for example, a separate-pack type dental curable composition in which the ligand compound comprises a ligand containing a phosphorus atom.

Examples of the ligand containing a phosphorus atom include phosphine ligands, and phosphite ligands. Specific examples of the ligand containing a phosphorus atom include compounds represented by the following general formula (1), compounds represented by the following general formula (2), compounds represented by the following general formula (3), and compounds represented by the following general formula (4). The ligand containing a phosphorus atom may be used alone, or two or more thereof may be used in combination.

[Chem. 3]

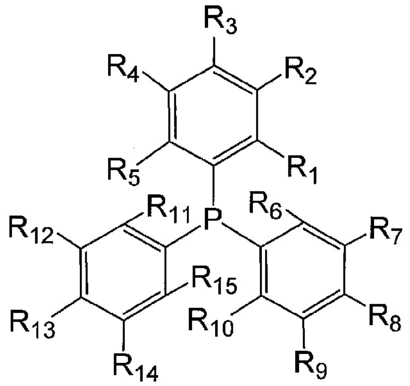

(1)

wherein $R_1$ to $R_{15}$ each independently represent a hydrogen atom, a halogen atom, a polar group, an optionally substituted alkyl group, or an optionally substituted alkoxy group.

[Chem. 4]

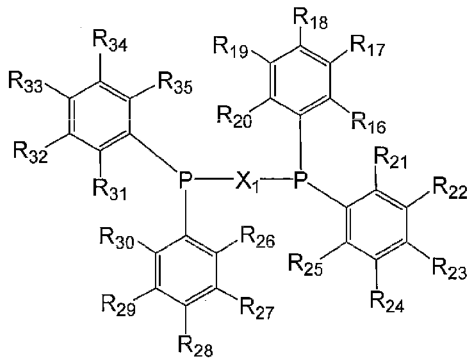

(2)

wherein $R_{16}$ to $R_{35}$ each independently represent a hydrogen atom, a halogen atom, a polar group, an optionally substituted alkyl group, or an optionally substituted alkoxy group, and $X_1$ represents an optionally substituted divalent aliphatic group.

[Chem. 5]

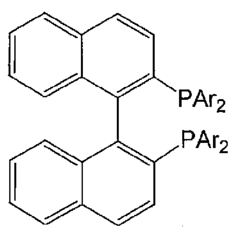

(3)

wherein Ar each independently represent a group represented by the following general formula (3-a).

[Chem. 6]

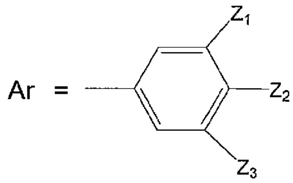

(3 − a)

wherein $Z_1$ to $Z_3$ each independently represent a hydrogen atom, a halogen atom, an optionally substituted alkyl group, or an optionally substituted alkoxy group, and at least one of $Z_1$ to $Z_3$ is a hydrogen atom.

$$P(OY_1)_3 \quad (4),$$

wherein $Y_1$ each independently represent an optionally substituted alkyl group, or an optionally substituted aryl group.

In view of curability, the mechanical strength of the cured product, and adhesive properties, particularly the bond durability to tooth structure and CAD/CAM resins, preferred among the ligands containing a phosphorus atom are compounds represented by the general formula (1), and compounds represented by the general formula (2).

Preferably, $R_1$ to $R_{15}$ are hydrogen atoms, optionally substituted alkyl groups, or optionally substituted alkoxy groups.

Preferably, $R_{16}$ to $R_{35}$ are hydrogen atoms, optionally substituted alkyl groups, or optionally substituted alkoxy groups.

Preferably, $Z_1$ to $Z_3$ are hydrogen atoms, optionally substituted alkyl groups, or optionally substituted alkoxy groups.

The optionally substituted alkyl groups represented by $R_1$ to $R_{15}$ may be linear or branched. The number of carbon atoms in the alkyl groups represented by $R_1$ to $R_{15}$ is not particularly limited, and is preferably 1 to 12, more preferably 1 to 6, even more preferably 1 to 4, particularly preferably 1 to 3.

Examples of the alkyl groups represented by $R_1$ to $R_{15}$ include methyl groups, ethyl groups, n-propyl groups, isopropyl groups, n-butyl groups, isobutyl groups, sec-butyl groups, tert-butyl groups, n-pentyl groups, isopentyl groups, sec-pentyl groups, neopentyl groups, n-hexyl groups, isohexyl groups, n-heptyl groups, n-octyl groups, n-nonyl groups, n-decyl groups, n-undecyl groups, and n-dodecyl groups. The alkyl groups represented by $R_1$ to $R_{15}$ may be unsubstituted.

Examples of the substituents on the alkyl groups represented by $R_1$ to $R_{15}$ include halogen atoms (a fluorine atom, a chlorine atom, a bromine atom, an iodine atom), hydroxyl groups, alkoxy groups having 1 to 6 carbon atoms, dialkylamino groups having 1 to 6 carbon atoms in each alkyl group, and amino groups.

Examples of the halogen atoms represented by $R_1$ to $R_{15}$ include a fluorine atom, a chlorine atom, a bromine atom, and an iodine atom.

Examples of the polar groups represented by $R_1$ to $R_{15}$ include acid anhydride groups, carboxylic acid groups, carboxylic acid ester groups, carboxylic acid chloride groups, carboxylic acid amide groups, carboxylate groups, sulfonic acid groups, sulfonic acid ester groups, sulfonyl chloride groups, sulfonic acid amide groups, sulfonate groups, aldehyde groups, epoxy groups, cyano groups, amino groups, monoalkyl-substituted amino groups, dialkyl-substituted amino groups, imide groups, and oxazoline groups. In view of curability and the mechanical strength of the cured product, preferred are carboxylic acid groups, carboxylic acid ester groups, carboxylic acid chloride groups, carboxylic acid amide groups, carboxylate groups, sulfonic acid groups, sulfonic acid ester groups, sulfonyl chloride groups, sulfonic acid amide groups, sulfonate groups, and aldehyde groups, more preferably carboxylic acid groups, carboxylic acid ester groups, carboxylic acid chloride groups, carboxylate groups, sulfonic acid groups, sulfonic acid ester groups, sulfonyl chloride groups, sulfonate groups, and aldehyde groups, even more preferably carboxylic acid groups, carboxylic acid ester groups, carboxylic acid chloride groups, carboxylate groups, sulfonic acid groups, sulfonic acid ester groups, sulfonyl chloride groups, and sulfonate groups. Examples of salts of carboxylate groups and sulfonate groups include alkali metal salts such as lithium salts, sodium salts, and potassium salts; and alkali-earth metal salts such as magnesium salts, calcium salts, strontium salts, barium salts, and radium salts.

When $R_1$ to $R_{15}$ are polar groups, the number of polar groups is preferably 1 to 9, more preferably 1 to 5, even more preferably 1 to 3.

When $R_1$ to $R_{15}$ are alkyl groups having a substituent, trifluoromethyl groups represent a specific example of such alkyl groups.

The optionally substituted alkoxy groups represented by $R_1$ to $R_{15}$ may be linear or branched. The number of carbon atoms in the alkoxy groups represented by $R_1$ to $R_{15}$ is not particularly limited, and is preferably 1 to 12, more preferably 1 to 6, even more preferably 1 to 4, particularly preferably 1 to 3.

Examples of the alkoxy groups represented by $R_1$ to $R_{15}$ include methoxy groups, ethoxy groups, n-propoxy groups, isopropoxy groups, n-butoxy groups, sec-butoxy groups, tert-butoxy groups, n-pentyloxy groups, isopentyloxy groups, sec-pentyloxy groups, tert-pentyloxy groups, neopentyloxy groups, n-hexyloxy groups, isohexyloxy groups, sec-hexyloxy groups, tert-hexyloxy groups, and neohexyloxy groups.

Examples of the substituents on the alkoxy groups represented by $R_1$ to $R_{15}$ include the same substituents as those of the alkyl groups represented by $R_1$ to $R_{15}$.

$R_1$ to $R_{15}$ may be the same or different. For example, $R_1$ to $R_{15}$ may partly represent the same hydrogen atoms, alkyl groups, or alkoxy groups.

The optionally substituted alkyl groups represented by $R_{16}$ to $R_{35}$ are the same as the optionally substituted alkyl groups represented by $R_1$ to $R_{15}$. The optionally substituted alkoxy groups represented by $R_{16}$ to $R_{35}$ are the same as the optionally substituted alkoxy groups represented by $R_1$ to $R_{15}$. The halogen atoms represented by $R_{16}$ to $R_{35}$ are the same as the halogen atoms represented by $R_1$ to $R_{15}$. The polar groups represented by $R_{16}$ to $R_{35}$ are the same as the polar groups represented by $R_1$ to R15.

The optionally substituted divalent aliphatic group represented by $X_1$ may be linear or branched.

The divalent aliphatic group has preferably 1 to 20 carbon atoms, more preferably 1 to 16 carbon atoms, even more preferably 1 to 12 carbon atoms, particularly preferably 1 to 8 carbon atoms.

Examples of the divalent aliphatic group represented by $X_1$ include alkylene groups, alkenylene groups, and alkynylene groups. Preferred are alkylene groups.

Examples of the alkylene groups include methylene groups, ethylene groups, propylene groups, butylene groups, methylpropylene groups, dimethylpropylene groups, pentamethylene groups, hexamethylene groups, heptamethylene groups, octamethylene groups, nonamethylene groups, decamethylene groups, undecamethylene groups, and dodecamethylene groups.

Examples of the substituents on the divalent aliphatic group represented by $X_1$ include the same substituents as those of the alkyl groups represented by $R_1$ to $R_{15}$.

In formula (3), the multiple Ar may be the same or different. In groups represented by general formula (3-a), $Z_1$ to $Z_3$ may be the same or different. The optionally substituted alkyl groups represented by $Z_1$ to $Z_3$ are the same as the optionally substituted alkyl groups represented by $R_1$ to $R_{15}$. At least one of $Z_1$ to $Z_3$ is a hydrogen atom, and $Z_1$ to $Z_3$ may all be hydrogen atoms. Specific examples of Ar include the following groups.

[Chem. 7]

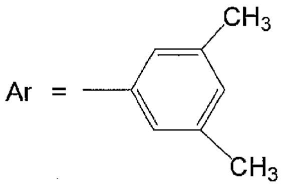

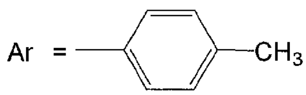

In certain embodiments, one or two of $Z_1$ to $Z_3$ are hydrogen atoms, and the remaining one or two of $Z_1$ to $Z_3$ may be a linear or branched C1 to C6 alkyl group(s) substituted with a halogen atom, a linear or branched C1 to C4 alkyl group(s) substituted with a fluorine atom, or a trifluoromethyl group. The optionally substituted alkoxy groups represented by $Z_1$ to $Z_3$ are the same as the optionally substituted alkoxy groups represented by $R_1$ to $R_{15}$. A certain preferred embodiment is, for example, a phosphine compound in which Ar in compounds represented by general formula (3) are all 3,5-dimethylphenyl groups. Another preferred embodiment is, for example, a phosphine compound in which Ar in compounds represented by general formula (3) are all 4-methylphenyl groups.

In phosphite ligands represented by general formula (4), the three $Y_1$ may be the same or different. The optionally substituted alkyl groups represented by $Y_1$ are the same as the optionally substituted alkyl groups represented by $R_1$ to $R_{15}$. The optionally substituted aryl groups represented by $Y_1$ have preferably 6 to 20 carbon atoms, more preferably 6 to 14 carbon atoms, even more preferably 6 to 10 carbon atoms. Examples of the substituents on the aryl groups represented by $Y_1$ include the same substituents as those of the alkyl groups represented by $R_1$ to $R_{15}$. Examples of the optionally substituted aryl groups represented by $Y_1$ include phenyl groups, biphenyl groups, indenyl groups, naphthyl groups, anthryl groups, phenanthryl groups, fluorenyl groups, and pyrenyl groups; and alkyl-substituted phenyl groups such as tolyl groups, xylyl groups, trimethylphenyl groups, ethylphenyl groups, isopropylphenyl groups, and tetramethylphenyl groups. A certain preferred embodiment is, for example, a phosphite compound in which the three $Y_1$ are 1,1,1,3,3,3-hexafluoro-2-propyl groups. Another preferred embodiment is, for example, a phosphite compound in which the three $Y_1$ are 2,4,-di-tert-butylphenyl groups.

Examples of the monodentate phosphine compounds represented by general formula (1) include phosphine

compounds having an electron donating group, such as triphenylphosphine, diphenyl(o-tolyl)phosphine, tri(o-tolyl)phosphine, tri(p-tolyl)phosphine, tris(2,4,6-trimethylphenyl)phosphine, tris(2,6-dimethylphenyl)phosphine, tris(2-methoxyphenylphosphine), tris(4-methoxyphenylphosphine), tris(2,6-dimethoxyphenyl)phosphine (hereinafter, also referred to by the abbreviation "DMPP"), diphenyl(2-methoxyphenyl)phosphine, and 4-(dimethylamino)triphenylphosphine; and phosphine compounds having an electron withdrawing group, such as (2-fluorophenyl)diphenylphosphine, (2-chlorophenyl)diphenylphosphine, (2-bromophenyl)diphenylphosphine, (pentafluorophenyl)diphenylphosphine, bis(pentafluorophenyl)phenylphosphine (hereinafter, also referred to by the abbreviation "BPFPP"), tris(pentafluorophenyl)phosphine (hereinafter, also referred to by the abbreviation "TPFPP"), tris(4-fluorophenyl)phosphine (hereinafter, also referred to by the abbreviation "TFPP"), tris(4-chlorophenyl)phosphine, tris(4-bromophenyl)phosphine, tris(4-trifluoromethylphenyl)phosphine, tris(4-carboxyphenyl)phosphine, sodium diphenylphosphinobenzene-3-sulfonate, and trisodium triphenylphosphine-3,3',3"-trisulfonate.

Examples of the bidentate phosphine compounds represented by general formula (2) include phosphine compounds such as bis(diphenylphosphino)methane , 1,2-bis(diphenylphosphino)ethane, 1,3-bis(diphenylphosphino)propane, 1,4-bis(diphenylphosphino)butane, 1,5-bis(diphenylphosphino)pentane, and 1,6-bis(diphenylphosphino)hexane.

Examples of the bidentate phosphine compounds represented by general formula (3) include (±)-2,2'-bis(diphenylphosphino)-1,1'-binaphthyl (hereinafter, also referred to by the abbreviation "BINAP"), (±)-2,2'-bis(di-p-tolylphosphino)-1,1'-binaphthyl, (±)-2,2'-bis(di-p-fluorophosphino)-1,1'-binaphthyl, (±)-2,2'-bis(di-p-trifluoromethylphosphino)-1,1'-binaphthyl, and (±)-2,2'-bis[di(3,5-xylyl)phosphino]-1,1'-binaphthyl.

Examples of the phosphite compounds represented by general formula (4) include trimethylphosphite, triethylphosphite, tris(1,1,1,3,3,3-hexafluoro-2-propyl)phosphite, triphenyl phosphite, and tris(2,4-di-t-butylphenyl)phosphite.

Examples of the ligands containing a nitrogen atom include compounds represented by general formula (5), compounds represented by general formula (6), and a polydentate ligand (7) containing a nitrogen-containing heterocyclic ring. The ligands containing a nitrogen atom may be used alone, or two or more thereof may be used in combination.

$R_{36}R_{37}N\text{-}X_2\text{-}NR_{38}R_{39}$ (5),

wherein $R_{36}$ to $R_{39}$ each independently represent an optionally substituted alkyl group, and $X_2$ represents an optionally substituted divalent aliphatic group.

[Chem. 8]

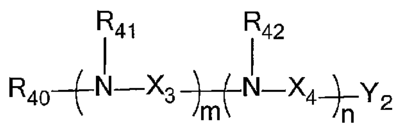

(6)

wherein $R_{40}$, $R_{41}$, and $R_{42}$ each independently represent an optionally substituted alkyl group, $X_3$ and $X_4$ each independently represent an optionally substituted divalent aliphatic group that may contain an oxygen atom and/or a nitrogen atom, m and n each independently represent an integer of 1 or more, $Y_2$ represents an optionally substituted monoalkylamino group or dialkylamino group, and any two or more of $R_{40}$, $R_{41}$, $R_{42}$, and $Y_2$ may together form a ring, and $R_{41}$, $R_{42}$, $X_3$, and $X_4$ may be the same or different when $R_{41}$, $R_{42}$, $X_3$, and $X_4$ are plural.

The optionally substituted alkyl groups represented by $R_{36}$ to $R_{39}$ are the same as the optionally substituted alkyl groups represented by $R_1$ to $R_{15}$.

The optionally substituted divalent aliphatic groups represented by $X_2$ are the same as the optionally substituted divalent aliphatic groups represented by $X_1$.

The optionally substituted alkyl groups represented by $R_{40}$, $R_{41}$, and $R_{42}$ are the same as the optionally substituted alkyl groups represented by $R_1$ to $R_{15}$. The number of carbon atoms in the monoalkylamino group ($-NHR^a$ ($R^a$ represents an alkyl group)) and dialkylamino group ($-NR^bR^c$ ($R^b$ and $R^c$ represent alkyl groups)) represented by $Y_2$ is not particularly limited, and is preferably 1 to 12, more preferably 1 to 6, even more preferably 1 to 4, particularly preferably 1 to 3. Examples of the alkyl groups in the monoalkylamino group and dialkylamino group represented by $Y_2$ include the optionally substituted alkyl groups represented by $R_1$ to $R_{15}$ satisfying the foregoing carbon counts. The dialkylamino group may be one having the foregoing carbon counts in each alkyl group. Examples of the optionally substituted monoalkylamino group represented by $Y_2$ include methylamino groups, ethylamino groups, propylamino groups, isopropylamino groups, butylamino groups, isobutylamino groups, t-butylamino groups, pentylamino groups, and hexylamino groups. Examples of the optionally substituted dialkylamino group represented by $Y_2$ include dimethylamino groups, diethylamino groups, dipropylamino groups, diisopropylamino groups, dibutylamino groups, diisobutylamino groups,

dipentylamino groups, dihexylamino groups, and ethylmethylamino groups. The alkyl groups in the monoalkylamino group and dialkylamino group represented by $Y_2$ may be substituted with a substituent. Examples of the substituent include the same substituents as those of the alkyl groups represented by $R_1$ to $R_{15}$.

The divalent aliphatic groups represented by $X_3$ and $X_4$ may be linear or branched. The divalent aliphatic groups have preferably 1 to 20 carbon atoms, more preferably 1 to 16 carbon atoms, even more preferably 1 to 12 carbon atoms, particularly preferably 1 to 8 carbon atoms. Examples of the divalent aliphatic groups include alkylene groups, alkenylene groups, and alkynylene groups. preferred are alkylene groups. Examples of the alkylene groups include methylene groups, ethylene groups, propylene groups, butylene groups, methylpropylene groups, dimethylpropylene groups, pentamethylene groups, hexamethylene groups, heptamethylene groups, octamethylene groups, nonamethylene groups, decamethylene groups, undecamethylene groups, and dodecamethylene groups. Examples of the substituents on the divalent aliphatic groups represented by $X_3$ and $X_4$ include the same substituents as those of the divalent aliphatic group represented by $X_1$. The divalent aliphatic groups represented by $X_3$ and $X_4$ may contain an oxygen atom and/or a nitrogen atom. $X_3$ and $X_4$ may be the same or different.

The symbols m and n each independently represent an integer of 1 or more, preferably an integer of 1 to 8, more preferably an integer of 1 to 6, even more preferably an integer of 1 to 5, particularly preferably an integer of 1 to 3. The symbols m and n may be the same or different.

Any two or more of $R_{40}$, $R_{41}$, $R_{42}$, and $Y_2$ may together form a ring. For example, $R_{40}$, $R_{41}$, or $R_{42}$ may form a ring with $Y_2$. Alternatively, $R_{40}$ and $Y_2$, and $R_{41}$ and $R_{42}$ may separately form a ring, creating a compound with two rings. Furthermore, the nitrogen atom in the amino group represented by $Y_2$ may form a ring with $R_{40}$. The ring may contain an oxygen atom and/or a nitrogen atom. In certain embodiments, compounds represented by general formula (6) may be compounds having a bicyclic ring. For example, in other embodiments, compounds represented by general formula (6) may be compounds having a bicyclic ring, with a ring formed by $Y_2$ and $R_{40}$, and another ring formed by $R_{41}$ and $Y_2$ or $R_{42}$ and $Y_2$.

In a certain preferred embodiment, $R_{40}$, $R_{41}$, and $R_{42}$ are linear or branched C1 to C6 alkyl groups that may have a substituent.

Another certain preferred embodiment is, for example, a separate-pack type dental curable composition in which at least one of the first and second agents comprises a ligand compound, and the ligand compound is at least one selected from the group consisting of a compound represented by general formula (1), and a compound represented by general formula (5).

Yet another certain preferred embodiment is, for example, a separate-pack type dental curable composition in which at least one of the first and second agents comprises a ligand compound, and the ligand compound is a compound represented by general formula (6), wherein, in the compound represented by general formula (6), $R_{40}$, $R_{41}$, and $R_{42}$ represent linear or branched C1 to C6 alkyl groups that may have a substituent, the divalent aliphatic groups represented by $X_3$ and $X_4$ represent alkylene groups containing no oxygen atom and no nitrogen atom, m and n each independently represent an integer of 1 or more, $Y_2$ represents an optionally substituted monoalkylamino group or dialkylamino group, and $R_{40}$, $R_{41}$, or $R_{42}$ forms a ring with $Y_2$.

Still another certain preferred embodiment is, for example, a separate-pack type dental curable composition in which at least one of the first and second agents comprises a ligand compound, and the ligand compound is a compound represented by general formula (6), wherein, in the general formula, m is 1 and n is 2, creating a compound containing four nitrogen atoms as a whole.

The polydentate ligand (7) containing a nitrogen-containing heterocyclic ring represents a bidentate or higher dentate ligand compound that comprises a heterocyclic ring containing a five-membered or six-membered ring having a nitrogen atom, and has two or more nitrogen atoms within the molecule. The polydentate ligand (7) has two or more nitrogen atoms within the molecule, and may have three or more nitrogen atoms within the molecule. The polydentate ligand (7) may contain one heterocyclic ring, or two or more heterocyclic rings. Examples of the nitrogen-containing heterocyclic ring include nitrogen-containing five-membered rings such as a pyrrole ring, a pyrazole ring, and an imidazole ring; and a nitrogen-containing six-membered rings such as a pyridine ring, a pyrazine ring, a pyridazine ring, a piperazine ring, a pyrimidine ring, and a triazine ring. The nitrogen-containing heterocyclic ring may be a fused ring formed by the nitrogen atom-containing five-membered or six-membered ring with other rings (for example, an aromatic ring), or a fused ring formed by nitrogen atom-containing five-membered or six-membered rings. Examples of the fused rings formed by the nitrogen atom-containing five-membered or six-membered ring with an aromatic ring include a quinoline ring, an isoquinoline ring, an indole ring, a benzoimidazole ring, and a benzotriazole ring. The polydentate ligand (7) comprises a heterocyclic ring containing a nitrogen atom-containing five-membered or six-membered ring. An example is a ligand compound that comprises a fused ring, such as an indole ring, a benzoimidazole ring, or a benzotriazole ring, and a heterocyclic ring containing a nitrogen atom-containing five-membered or six-membered ring. Concerning denticity, the polydentate ligand (7) is a bidentate or higher dentate ligand compound, and may be tridentate or tetradentate, for example.

Examples of polydentate amine compounds represented by the general formula (5) include two-coordinate

polydentate amine compounds such as N,N,N',N'-tetramethylethylenediamine (hereinafter, also referred to by the abbreviation "TMEDA"), N,N,N',N'-tetramethylpropylenediamine (hereinafter, also referred to by the abbreviation "TMPDA"), N,N,N',N'-tetramethyl-1,4-diaminobutane, N,N,N',N'-tetraethylethylenediamine (hereinafter, also referred to by the abbreviation "TEEDA"), and N,N,N',N'-tetrakis(2-hydroxyethyl)ethylenediamine.

Examples of compounds represented by the general formula (6) include polydentate amine compounds, for example, compounds having a cyclic ring, such as 1,4,8,11-tetramethyl-1,4,8,11-tetraazacyclotetradecane, compounds having a bicyclic ring such as 4,11-dimethyl-1,4,8,11-tetraazabicyclohexadecane, and compounds with no rings, such as 2,5,9,12-tetramethyl-2,5,9,12-tetraazatetradecane, 2,6,9,13-tetramethyl-2,6,9,13-tetraazatetradecane, 2,5,8,12-tetramethyl-2,5,8,12-tetraazatetradecane, N,N,N,N',N",N"-pentamethyldiethylenetriamine (hereinafter, also referred to by the abbreviation "PMDETA"), hexamethyltris(2-aminoethyl)amine, N,N-bis(2-dimethylaminoethyl)-N,N'-dimethylethylenediamine (hereinafter, also referred to by the abbreviation "HMTETA"), and tris[2-(dimethylamino)ethyl]amine (hereinafter, also referred to by the abbreviation "$Me_6$TREN").

Examples of the polydentate ligand (7) containing a nitrogen-containing heterocyclic ring include 2,2-bipyridine, 4,4'-di-(5-nonyl)-2,2'-bipyridine, N-(n-propyl)pyridylmethane imine, N-(n-octyl)pyridylmethane imine, N-propyl-N,N-di(2-pyridylmethyl)amine, N',N"-dimethyl-N',N"-bis((pyridin-2-yl)methyl)ethane-1,2-diamine, 2,6-bis(1-pyrazole)-pyridine (hereinafter, also referred to by the abbreviation "DPP"), 2-(2-pyridyl)benzoimidazole, tris[(2-pyridyl)methyl]amine, 3,6-di(2-pyridyl)-1,2,4,5-tetrazine, N,N,N',N'-tetrakis(2-pyridylmethyl)ethylenediamine, and 2,4,6-tri(2-pyridyl)-1,3,5-triazine.

Preferred for use among these are tri(o-tolyl)phosphine, tris(2,6-dimethoxyphenyl)phosphine, triphenyl phosphite, TMEDA, TMPDA, TEEDA, PMDETA, and $Me_6$TREN. The ligand compound may be incorporated alone, or two or more thereof may be incorporated in combination. The ligand compound is used to enhance the catalytic activity of the transition metal compound in a separate-pack type dental curable composition of the present invention. In view of curability, the mechanical strength of the cured product, and adhesive properties, particularly the bond durability to tooth structure and CAD/CAM resins, the content of the ligand compound ranges preferably from 0.005 to 10 parts by mass, more preferably from 0.01 to 5 parts by mass, even more preferably from 0.05 to 3 parts by mass with respect to total 100 parts by mass of polymerizable monomer components in a separate-pack type dental curable composition of the present invention.

A separate-pack type dental curable composition of the present invention comprises a redox-type polymerization initiator. However, in order to provide a dual-cure composition also capable of polymerizing upon exposure to light, a known photopolymerization initiator may optionally be incorporated in at least one of the first and second agents in a separate-pack type dental curable composition of the present invention, separately from the polymerization initiator system described above.

Examples of the photopolymerization initiator include α-diketones, ketals, thioxanthones, (bis)acylphosphine oxides, and α-aminoacetophenones.

Examples of the α-diketones include dl-camphorquinone (commonly known as CQ), benzyl, and 2,3-pentanedione.

Examples of the ketals include benzyl dimethyl ketal, and benzyl diethyl ketal.

Examples of the thioxanthones include 2-chlorothioxanthone, and 2,4-diethylthioxanthone.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, 2,4,6-trimethylbenzoylphenylphosphine oxide, 2,4,6-trimethylbenzoylmethoxyphenylphosphine oxide, 2,4,6-trimethylbenzoylethoxyphenylphosphine oxide, benzoylbis(2,6-dimethylphenyl)phosphine oxide, the water-soluble acylphosphine oxide compounds disclosed in JP H03-57916 B, and salts of these (for example, sodium salts, potassium salts, ammonium salts).

Examples of bisacylphosphine oxides include bis(2,4,6-trimethylbenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, dibenzoylphenylphosphine oxide, tris(2,4-dimethylbenzoyl)phosphine oxide, tris(2-methoxybenzoyl)phosphine oxide, and salts of these (for example, sodium salts, potassium salts, ammonium salts). Preferred among these (bis)acylphosphine oxides are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the α-aminoacetophenones include 2-benzyl-2-dimethylamino-1-(4-morpholinophenyl)-1-butanone, 2-benzyl-2-diethylamino-1-(4-morpholinophenyl)-1-butanone, 2-benzyl-2-dimethylamino-1-(4-morpholinophenyl)-1-propanone, 2-benzyl-2-diethylamino-1-(4-morpholinophenyl)-1-propanone, 2-benzyl-2-dimethylamino-1-(4-morpholinophenyl)-1-pentanone, and 2-benzyl-2-diethylamino-1-(4-morpholinophenyl)-1-pentanone.

The photopolymerization initiator may be used alone, or two or more thereof may be used in combination.

The content of the photopolymerization initiator is not particularly limited. However, in view of the curability and other properties of the dental curable composition obtained, the content of the photopolymerization initiator is preferably

0.001 to 10 parts by mass, more preferably 0.005 to 5 parts by mass, even more preferably 0.01 to 3 parts by mass with respect to total 100 parts by mass of the polymerizable monomer components.

For enhanced photocurability, the photopolymerization initiator used in a separate-pack type dental curable composition of the present invention may be used with polymerization accelerators for use with photopolymerization initiators.

It is also possible to use the organic peroxide (D) with polymerization accelerators.

Examples of polymerization accelerators that can be used with organic peroxide (D) and/or photopolymerization initiators include tertiary amines, aldehydes, thiol compounds, triazine compounds substituted with a trihalomethyl group, thiourea compounds, sulfinic acids, benzotriazole compounds, benzoimidazole compounds, sulfites, bisulfites, borate compounds, and barbituric acid and its derivatives. The polymerization accelerators may be used alone, or two or more thereof may be used in combination.

Examples of the tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N, N-bis(2-hydroxyethyl)-3,5-diisopropylaniline, N,N-bis(2-hydroxyethyl)-3,5-di-t-butylaniline, n-butoxyethyl 4-(N,N-dimethylamino)benzoate, 2-(methacryloyloxy)ethyl 4-(N,N-dimethylamino)benzoate, ethyl 4-(N,N-dimethylamino)benzoate, butyl 4-(N,N-dimethylamino)benzoate, N-methyldiethanolamine, 4-(N,N-dimethylamino)benzophenone, trimethylamine, triethylamine, N-methyldiethanolamine, N-ethyldiethanolamine, N-n-butyldiethanolamine, N-lauryldiethanolamine, triethanolamine, 2-(dimethylamino)ethyl (meth)acrylate, N-methyldiethanolamine di(meth)acrylate, N-ethyldiethanolamine di(meth)acrylate, triethanolamine mono(meth)acrylate, triethanolamine di(meth)acrylate, and triethanolamine tri(meth)acrylate.

Examples of the aldehydes include terephthalaldehyde, and benzaldehyde derivatives. Examples of the benzaldehyde derivatives include dimethylaminobenzaldehyde, p-methoxybenzaldehyde, p-ethoxybenzaldehyde, and p-n-octyloxybenzaldehyde. Examples of the thiol compounds include 3-mercaptopropyltrimethoxysilane, 2-mercaptobenzoxazole, 2-mercaptobenzoimidazole, decanethiol, and thiobenzoic acid. The triazine compounds substituted with a trihalomethyl group may be any known compounds, provided that it is an s-triazine compound with at least one trihalomethyl group such as a trichloromethyl group, and a tribromomethyl group.

Examples of the thiourea compounds include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea, and 4,4-dimethylethylenethiourea. The thiourea compounds may be used alone, or two or more thereof may be used in combination.

Examples of the sulfinic acids include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Preferred among these are sodium benzenesulfinate, sodium p-toluenesulfinate, 2,4,6-triisopropylbenzenesulfinic acid, and sodium 2,4,6-triisopropylbenzenesulfinate. The sulfinic acids may be used alone, or two or more thereof may be used in combination.

Examples of the benzotriazole compounds include 1H-benzotriazole (hereinafter, also referred to by the abbreviation "BTA"), 5-methyl-1H-benzotriazole, and 5,6-dimethyl-1H-benzotriazole.

Examples of the benzoimidazole compounds include benzoimidazole, 5-methylbenzoimidazole, and 5,6-dimethylbenzoimidazole.

Examples of the sulfites include sodium sulfite, potassium sulfite, calcium sulfite, and ammonium sulfite.

Examples of the bisulfites include sodium bisulfite, and potassium bisulfite.

Examples of the borate compounds include arylborate compounds having 1 to 4 aryl groups per molecule (for example, tetraphenylboron, tetrakis(p-chlorophenyl)boron), ad salts of these.

Examples of the barbituric acid and its derivatives include barbituric acid, 5-butylbarbituric acid, 1 ,3,5-trimethylbarbituric acid, 1-cyclohexyl-5-ethylbarbituric acid, 1-benzyl-5-phenylbarbituric acid, and salts of these.

The polymerization accelerators for photopolymerization initiators may be used alone, or two or more thereof may be used in combination. The content of polymerization accelerators for photopolymerization initiators is not particularly limited. However, in view of the curability and other properties of the dental curable composition obtained, the content of polymerization accelerators for photopolymerization initiators is preferably 0.001 to 10 parts by mass, more

preferably 0.005 to 5 parts by mass, even more preferably 0.01 to 3 parts by mass with respect to total 100 parts by mass of polymerizable monomer components in a separate-pack type dental curable composition of the present invention.

A dental curable composition of the present invention may additionally comprise a fluorine-ion releasing substance in at least one of the first and second agents. By incorporating a fluorine-ion releasing substance, a dental resin cement can be obtained that can impart acid resistance to tooth structure.

Examples of the fluorine-ion releasing substance include fluorine-ion releasing polymers, such as copolymers of methyl methacrylate and methacrylic acid fluoride; hydrofluorides of primary, secondary, or tertiary amines of aliphatic or alicyclic nature, such as cetylamine hydrofluoride, cyclohexylamine hydrofluoride, diisobutylamine hydrofluoride, and triethylamine trihydrofluoride; and metal fluorides such as sodium fluoride, potassium fluoride, sodium monofluorophosphate, lithium fluoride, and ytterbium fluoride. The fluorine-ion releasing substance may be used alone, or two or more thereof may be used in combination.

A separate-pack type dental curable composition of the present invention may comprise a pH adjuster in at least one of the first and second agents. The pH adjuster is used to adjust and stabilize the pH of a separate-pack type dental curable composition of the present invention.

The pH adjuster is not particularly limited, as long as the present invention can exhibit its effects. Preferred for use are acids such as lactic acid, succinic acid, gluconic acid, citric acid, phosphoric acid, and carbonic acid, and salts of these. The pH adjuster may be used alone, or two or more thereof may be used in combination.

Examples of the phosphates include:

alkali metal salts of phosphate, such as trisodium phosphate, and tripotassium phosphate;
alkali metal salts of hydrogen phosphate, such as disodium hydrogen phosphate, and dipotassium hydrogen phosphate;
alkali metal salts of dihydrogen phosphate, such as sodium dihydrogen phosphate, and potassium dihydrogen phosphate;
alkali metal salts of alkyl phosphate, such as sodium dodecylphosphate;
sodium glycerophosphate, and disodium glycerophosphate;
alkali-earth metal salts of phosphate, such as tricalcium phosphate, and trimagnesium phosphate;
alkali-earth metal salts of hydrogen phosphate, such as calcium hydrogen phosphate, and magnesium hydrogen phosphate; and
alkali-earth metal salts of dihydrogen phosphate, such as calcium dihydrogen phosphate.

Preferred for use among these are disodium hydrogen phosphate, sodium dodecylphosphate, sodium glycerophosphate, and disodium glycerophosphate.

In a separate-pack type dental curable composition of the present invention, it is also possible to incorporate additives such as polymerization inhibitors, ultraviolet absorbers, thickeners, solvents (for example, water, organic solvent), colorants, antimicrobial agents, and fragrances in at least one of the first and second agents, provided that such additives do not hinder the effectiveness of the present invention. These may be incorporated alone, or two or more thereof may be incorporated in combination.

Examples of the polymerization inhibitors include hydroquinone, hydroquinone monomethyl ether, dibutyl hydroquinone, dibutyl hydroquinone monomethyl ether, t-butylcatechol, 2-t-butyl-4,6-dimethylphenol, 2,6-di-t-butylphenol, and 2,6-di-t-butyl-4-methylphenol.

In certain embodiments, the content of a solvent (for example, water, organic solvent) in the separate-pack type dental curable composition is preferably less than 1 mass%, more preferably less than 0.1 mass%, even more preferably less than 0.01 mass% in a total amount of the separate-pack type dental curable composition.

A separate-pack type dental curable composition of the present invention can be prepared according to an ordinary method, depending on the type and amount of the aforementioned components. A separate-pack type dental curable composition of the present invention is used in a two-pack form. The two-pack form can be appropriately selected from various forms, such as powder and liquid, paste and liquid, and two pastes. In view of workability, two pastes are used in more preferred embodiments. Preferably, each paste is kept isolated from the other during storage, and the two pastes are kneaded immediately before use to initiate chemical polymerization for curing. Usually, the pastes are prepared by mixing a liquid component formulated with components other than filler (G), and kneading it with a powdery filler (G).

A separate-pack type dental curable composition of the present invention has use in bonding dental prostheses such as crowns, inlays, and bridges to tooth structures, as well as in the construction of abutments in defects in the affected tooth area. A separate-pack type dental curable composition of the present invention can be used as a dental cement such as a dental resin cement. Particularly preferred as such dental resin cements are self-adhesive dental resin cements.

The present invention encompasses embodiments combining the foregoing features in various ways within the technical idea of the present invention, provided that the present invention can exhibit its effects.

EXAMPLES

The following describes the present invention in greater detail using Examples and Comparative Examples. It is to be noted, however, that the present invention is not limited to the following. The abbreviations and symbols used hereafter are as follows. The compounds and fillers used in the following Examples and Comparative Examples are commercially available products, except where the production methods are specifically described.

[Polymerizable monomer (A) having an acidic group]
MDP: 10-methacryloyloxydecyl dihydrogen phosphate
[Polymerizable monomer (B) having no acidic group]

HEMA: 2-hydroxyethyl methacrylate
Bis-GMA: 2,2-bis[4-(2-hydroxy-3-methacryloyloxypropoxy)phenyl]propane
D-2.6E: 2,2-bis(4-methacryloyloxypolyethoxyphenyl)propane (with an average of 2.6 moles of ethoxy group added)
TEGDMA: triethylene glycol dimethacrylate

[Ascorbic acid compound (C)]

PA: ascorbyl palmitate
ANa: sodium L-ascorbate

[Organic peroxide (D)]

THP: 1,1,3,3-tetramethylbutyl hydroperoxide
BPB: t-butyl peroxybenzoate

[Transition metal compound (E)]

CuA: copper(II) acetate
VOAA: vanadyl(IV) acetylacetonate

[Silane coupling agent (F)]

8-MOS: 8-methacryloyloxyoctyltrimethoxysilane
11-MUS: 11-methacryloyloxyundecyltrimethoxysilane
11-MUES: 11-methacryloyloxyundecyltriethoxysilane

[Silane coupling agents other than silane coupling agent (F)]
γ-MPS: γ-methacryloyloxypropyltrimethoxysilane

[Filler (G)]

Surface-treated barium glass

A barium glass (manufactured by Esstech, product code E-3000) was pulverized with a ball mill to obtain a barium glass powder. The pulverized barium glass powder had an average particle diameter of 2.4 μm as measured by volume with a laser diffraction particle size distribution analyzer (Model SALD-2300, manufactured by Shimadzu Corporation). One-hundred parts by mass of the pulverized barium glass powder was surface treated with three parts by mass of γ-methacryloyloxypropyltrimethoxysilane by an ordinary method to obtain a surface-treated barium glass.
R972: particulate silica manufactured by Nippon Aerosil Co., Ltd. under the trade name Aerosil® R972; average particle diameter: 16 nm
Alumina: aluminum oxide manufactured by Nippon Aerosil Co., Ltd. under the trade name AEROXIDE® Alu C; average particle diameter: 13 nm

[Ligand compound]
$P(OPh)_3$: triphenyl phosphite
[Polymerization accelerator]

TPBSS: sodium 2,4,6-triisopropylbenzenesulfinate
DEPT: N,N-bis(2-hydroxyethyl)-p-toluidine
BTA: 1H-benzotriazole
DMETU: 4,4-dimethylethylenethiourea

[Polymerization inhibitor]
BHT: 2,6-di-t-butyl-4-methylphenol

(Examples1 to 9 and Comparative Examples 1 to 4)

The components, excluding the fillers and ascorbic acid compounds, listed in Tables 1 and 2 were mixed at ordinary temperature to prepare a uniform liquid component. Subsequently, this liquid component was kneaded with the ascorbic acid compounds and fillers to prepare separate-pack type dental curable compositions of Examples 1 to 9 and Comparative Examples 1 to 4.

Immediately following their production, these separate-pack type dental curable compositions were utilized to measure polymerization conversion rate at 32°C, as well as bond strength to CAD/CAM resin, according to the methods described below. Tables 1 and 2 present the formulation ratios (parts by mass) and the test results for these dental curable compositions.

[Polymerization Conversion Rate of Separate-Pack Type Dental Curable Composition at 32°C]

An ATR measurement device (diamond micro ATR machine, single bounce horizontal ATR SmartOrbit®, manufactured by ThermoFisher Scientific) was set up on the FT-IR measurement instrument (Fourier transform infrared spectrometer Nicolet 6700, manufactured by ThermoFisher Scientific). The measurement conditions included a measurement range from 4,000 $cm^{-1}$ to 650 $cm^{-1}$, an incident angle of 45°, a single scan, and the use of a diamond prism.

The dental curable composition resulting from mixing the first and second agents in each Example and Comparative Example was placed on the sample platform of the diamond micro ATR machine adjusted to 32°C. Real-time IR measurements were conducted to measure the spectra of the dental curable composition at different time intervals. The real-time IR measurement was started at the time when the dental curable composition was placed, immediately after mixing.

The spectral analysis conditions are as follows: the carbonyl bond peaks (C=O, 1,710 $cm^{-1}$), unaffected by polymerization, were denoted as a1 and a2 before and after polymerization, and were employed as reference points. The ratios of the areas (b1/a1, b2/a2) of the double bond peaks (C=C, 1,640 $cm^{-1}$; represented as b1 and b2 before and after polymerization) with respect to their respective reference points were used to calculate the polymerization conversion rate c (%). The polymerization conversion rate in the table represents the highest value observed during a 40-minute measurement period (n = 3).

$$\text{Polymerization conversion rate c (\%)} = \{1-(b2/a2)/(b1/a1)\} \times 100$$

The polymerization conversion rate at 32°C is preferably 45% or higher, more preferably 47.5% or higher, even more preferably 50% or higher in view of adhesive properties, particularly the bond durability to the CAD/CAM resin, which possesses a few unreacted polymerizable groups on its surface.

[Tensile Bond Strength of Separate-Pack Type Dental Curable Composition to CAD/CAM Resin]

The CAD/CAM resin (manufactured by Kuraray Noritake Dental Inc. under the trade name KATANAAvencia Block) was polished with #1000 silicon carbide paper under running water. After polishing, the surface was dried by blowing air and removing the surface water. Once dry, the surface was subjected to sandblasting using a 50-micron alumina abrasive (manufactured by J. Morita Corp.) to create a surface prepared for adhesion. Subsequently, an adhesive tape having a thickness of about 150 μm with a round hole measuring 5 mm in diameter was attached to the prepared adherend surface to define a bonding area.

The dental curable composition prepared by mixing the first and second agents in each Example and Comparative Example was then applied onto one end surface (with a circular cross section) of a cylindrical stainless steel rod (7 mm in diameter, 2.5 cm in length).

Subsequently, the end surface with the dental curable composition was placed on the smooth surface (prepared adherend surface) within the circular hole, with the center of the circular hole approximately aligned with the center of the cylindrical stainless steel rod. The cylindrical stainless steel rod was then pressed perpendicularly against the smooth

surface to effect bonding and prepare a test sample. A total of ten test samples were prepared. After removing any excess dental curable composition that had protruded from the periphery of the cylindrical stainless steel rod during compression, the test samples were left at room temperature for 30 minutes, and immersed in distilled water. The test samples were then left for 24 hours in the distilled water, inside a thermostatic chamber held at 37°C. After an additional 10 days in the thermostatic chamber maintained at 70°C, the tensile bond strength was assessed to evaluate the bond durability.

The tensile bond strength was measured using a universal testing machine (manufactured by Shimadzu Corporation) with the crosshead speed set at 2 mm/min. The table presents the tensile bond strength as an average of measured tensile bond strength values from ten test samples.

[Table 1]

| Components (parts by mass) | | | Ex. 1 | Ex. 2 | Ex. 3 | Ex. 4 | Ex. 5 | Ex. 6 | Ex. 7 | Ex. 8 | Ex. 9 |
|---|---|---|---|---|---|---|---|---|---|---|---|
| First agent | Polymerizable monomer (A) having an acidic group | MDP | 20 | 20 | 20 | 20 | 20 | 20 | 20 | 20 | 20 |
| | Polymerizable monomer (B) having no acidic group | HEMA | 15 | 15 | 15 | 15 | 15 | 15 | 15 | 15 | 15 |
| | | Bis-GMA | 40 | 40 | 40 | 40 | 40 | 40 | 40 | 40 | 40 |
| | | D-2.6E | 25 | 25 | 25 | 25 | 25 | 25 | 25 | 25 | 25 |
| | Ascorbic acid compound (C) | PA | | | | | | | 3 | 3 | |
| | | ANa | | | | | | | | | |
| | Organic peroxide (D) | THP | 3 | 3 | 3 | 3 | 3 | 3 | | | 3 |
| | | BPB | | | | | | | | | |
| | Transition metal compound (E) | CuA | 0.02 | 0.02 | 0.02 | 0.03 | 0.04 | 0.02 | | | |
| | | VOAA | | | | | | | | | |
| | Polymerization inhibitor | BHT | 0.2 | 0.2 | 0.2 | 0.2 | 0.2 | 0.2 | 0.2 | 0.2 | 0.2 |
| | Filler (G) | Surface-treated barium glass | 220 | 220 | 220 | 220 | 220 | 220 | 220 | 220 | 220 |
| | | R972 | 10 | 10 | 10 | 10 | 10 | 10 | 10 | 10 | 10 |

(continued)

| Components (parts by mass) | | | Ex. 1 | Ex. 2 | Ex. 3 | Ex. 4 | Ex. 5 | Ex. 6 | Ex. 7 | Ex. 8 | Ex. 9 |
|---|---|---|---|---|---|---|---|---|---|---|---|
| Second agent | Polymerizable monomer (B) having no acidic group | D-2.6E | 80 | 80 | 80 | 80 | 80 | 80 | 80 | 80 | 80 |
| | | TEGDMA | | | | | | | | | |
| | Ascorbic acid compound (C) | PA | 3 | 3 | 3 | 3 | 3 | | | | 3 |
| | | ANa | | | | | | 3 | | | |
| | Organic peroxide (D) | THP | | | | | | | 3 | | |
| | | BPB | | | | | | | | 3 | |
| | Transition metal compound (E) | CuA | | | | | | | 0.02 | 0.02 | |
| | | VOAA | | | | | | | | | 0.02 |
| | Silane coupling agent (F) | 8-MOS | 20 | | | 20 | 20 | 20 | 20 | 20 | 20 |
| | | 11-MUS | | 20 | | | | | | | |
| | | 11-MUES | | | 20 | | | | | | |
| | Silane coupling agent other than (F) | γ-MPS | | | | | | | | | |
| | Polymerization accelerator | TPBSS | | | | | | | | | |
| | | DEPT | | | | | | | | | |
| | | BTA | | | | | | | | | |
| | | DMETU | | | | | | | | | |
| | Ligand compound | $P(OPh)_3$ | 3 | 3 | 3 | 3 | 3 | 3 | | | 3 |
| | Polymerization inhibitor | BHT | 0.1 | 0.1 | 0.1 | 0.1 | 0.1 | 0.1 | 0.1 | 0.1 | 0.1 |
| | Filler (G) | Surface-treated barium glass | 220 | 220 | 220 | 220 | 220 | 220 | 220 | 220 | 220 |
| | | R972 | | | | | | | | | |
| | | Alumina | 10 | 10 | 10 | 10 | 10 | 10 | 10 | 10 | 10 |
| Performance | Polymerization conversion rate at 32°C (%) | | 50 | 48 | 48 | 52 | 54 | 49 | 48 | 48 | 49 |
| | Tensile bond strength to CAD/CAM resin (MPa) | | 13.3 | 13.8 | 13.9 | 16.2 | 15.9 | 12.9 | 14.2 | 13.7 | 13.4 |

[Table 2]

| Components (parts by mass) | | | Com. Ex. 1 | Com. Ex. 2 | Com. Ex. 3 | Com. Ex. 4 |
|---|---|---|---|---|---|---|
| First agent | Polymerizable monomer (A) having an acidic group | MDP | 20 | 20 | 20 | 20 |
| | Polymerizable monomer (B) having no acidic group | HEMA | 15 | 15 | 15 | 15 |
| | | Bis-GMA | 40 | 40 | 40 | 40 |
| | | D-2.6E | 25 | 25 | 25 | 25 |
| | Ascorbic acid compound (C) | PA | | | | |
| | | ANa | | | | |
| | Organic peroxide (D) | THP | 3 | 3 | | 3 |
| | | BPB | | | 0.5 | |
| | Transition metal compound (E) | CuA | 0.02 | 0.02 | 0.001 | |
| | | VOAA | | | | |
| | Polymerization inhibitor | BHT | 0.2 | 0.2 | 0.15 | 0.2 |
| | Filler (G) | Surface-treated barium glass | 220 | 220 | 220 | 220 |
| | | R972 | 10 | 10 | 10 | 10 |
| Second agent | Polymerizable monomer (B) having no acidic group | D-2.6E | 80 | 80 | 80 | 80 |
| | | TEGDMA | 20 | | | |
| | Ascorbic acid compound (C) | PA | 3 | 3 | | |
| | | ANa | | | | |
| | Organic peroxide (D) | THP | | | | |
| | | BPB | | | | |
| | Transition metal compound (E) | CuA | | | | |
| | | VOAA | | | | 0.02 |
| | Silane coupling agent (F) | 8-MOS | | | 20 | 20 |
| | | 11-MUS | | | | |
| | | 11-MUES | | | | |
| | Silane coupling agent other than (F) | γ-MPS | | 20 | | |
| | Polymerization accelerator | TPBSS | | | 3 | |
| | | DEPT | | | 0.5 | |
| | | BTA | | | 3 | |
| | | DMETU | | | | 4 |
| | Ligand compound | $P(OPh)_3$ | 3 | 3 | | |
| | Polymerization inhibitor | BHT | 0.1 | 0.1 | 0.1 | 0.1 |
| | Filler (G) | Surface-treated barium glass | 220 | 220 | 220 | 220 |
| | | R972 | | | | |
| | | Alumina | 10 | 10 | 10 | 10 |

(continued)

| Components (parts by mass) | | Com. Ex. 1 | Com. Ex. 2 | Com. Ex. 3 | Com. Ex. 4 |
|---|---|---|---|---|---|
| Performance | Polymerization conversion rate at 32°C (%) | 49 | 51 | 38 | 39 |
| | Tensile bond strength to CAD/CAM resin (MPa) | 3.2 | 6.7 | 8.3 | 7.0 |

As can be seen from Table 1, the separate-pack type dental curable compositions of the present invention (Examples 1 to 9) showed superior results with a polymerization conversion rate of 48% to 54%, and a tensile bond strength of 12.9 MPa or higher to the CAD/CAM resin.

In contrast, as shown in Table 2, the separate-pack type dental curable compositions (Comparative Examples 1 and 2) containing no silane coupling agent (F) exhibited a low tensile bond strength of 6.7 MPa or less to the CAD/CAM resin, though a polymerization conversion rate of 49% to 51% was achieved with the use of a polymerization initiator system containing ascorbic acid compound (C).

In the separate-pack type dental curable compositions (Comparative Examples 3 and 4) containing no ascorbic acid compound (C), the polymerization conversion rate was 39% or less, and the tensile bond strength to CAD/CAM resin had a low value of 8.3 MPa or less, despite containing silane coupling agent (F). In Comparative Examples 3 and 4, the use of a polymerization accelerator in place of ascorbic acid compound (C) did not result in an elevation of either the polymerization conversion rate or the tensile bond strength to the CAD/CAM resin.

INDUSTRIAL APPLICABILITY

A separate-pack type dental curable composition of the present invention can be suitably used in applications such as bonding of dental prostheses, such as crowns, inlays, and bridges, to tooth structures, and construction of abutments in dental treatments.

## Claims

1. A separate-pack type dental curable composition comprising a polymerizable monomer (A) having an acidic group, a polymerizable monomer (B) having no acidic group, an ascorbic acid compound (C), an organic peroxide (D), a transition metal compound (E), and a silane coupling agent (F) represented by the following general formula (f-1),

[Chem. 1]

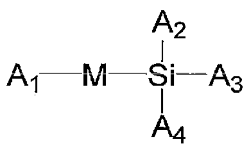

(f − 1)

wherein $A_1$ represents a polymerizable functional group selected from the group consisting of a (meth)acryloyloxy group, a vinyl group, and an epoxy group, M represents a divalent aliphatic group having a straight chain with a carbon chain length of 5 or more carbon atoms, or a divalent aromatic group having 6 or more carbon atoms, $A_2$, $A_3$, and $A_4$ each independently represent a hydroxyl group, a C1 to C5 alkyl group, or a C1 to C5 alkoxy group, and at least one of $A_2$, $A_3$,and $A_4$ is a C1 to C5 alkoxy group.

2. The separate-pack type dental curable composition according to claim 1, wherein the ascorbic acid compound (C) is at least one compound selected from the group consisting of a salt of ascorbic acid, and an ester of ascorbic acid.

3. The separate-pack type dental curable composition according to claim 1 or 2, wherein the transition metal compound (E) is a copper compound or a vanadium compound.

4. The separate-pack type dental curable composition according to any one of claims 1 to 3, which is separated into a first agent comprising the polymerizable monomer (A) having an acidic group, and a second agent comprising the silane coupling agent (F).

5. The separate-pack type dental curable composition according to any one of claims 1 to 4, wherein M is a divalent aliphatic group having a straight chain with a carbon chain length of 7 or more carbon atoms, or a divalent aromatic group having 7 or more carbon atoms.

6. The separate-pack type dental curable composition according to any one of claims 1 to 5, wherein $A_2$, $A_3$, and $A_4$ are each independently a hydroxyl group, a C1 to C3 alkyl group, or a C1 to C3 alkoxy group, and at least one of $A_2$, $A_3$, and $A_4$ is a C1 to C3 alkoxy group.

7. The separate-pack type dental curable composition according to any one of claims 1 to 6, wherein $A_2$, $A_3$, and $A_4$ are each independently a hydroxyl group, a methyl group, or a methoxy group, and at least one of $A_2$, $A_3$, and $A_4$ is a methoxy group.

8. The separate-pack type dental curable composition according to any one of claims 1 to 7, wherein $A_2$, $A_3$, and $A_4$ are methoxy groups.

9. The separate-pack type dental curable composition according to any one of claims 1 to 8, wherein M is an alkylene group having a straight chain with a carbon chain length of 8 or more carbon atoms.

10. The separate-pack type dental curable composition according to any one of claims 1 to 9, wherein the silane coupling agent (F) is at least one selected from the group consisting of 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, 8- (meth)acryloyloxyoctylmethyldimethoxysilane, 10-(meth)acryloyloxydecylmethyldimethoxysilane, 11-(meth)acryloyloxyundecylmethyldimethoxysilane, and (meth)acryloyloxymethylphenethyltrimethoxysilane.

11. The separate-pack type dental curable composition according to any one of claims 1 to 10, wherein the silane coupling agent (F) is at least one selected from the group consisting of 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, and (meth)acryloyloxymethylphenethyltrimethoxysilane.

12. The separate-pack type dental curable composition according to any one of claims 1 to 11, wherein the content of the ascorbic acid compound (C) and the content of the silane coupling agent (F) are 1:1 to 1:200 in terms of a mass ratio of ascorbic acid compound (C):silane coupling agent (F).

13. The separate-pack type dental curable composition according to any one of claims 1 to 12, which further comprises a filler (G).

14. The separate-pack type dental curable composition according to any one of claims 1 to 13, which further comprises a ligand compound.

15. The separate-pack type dental curable composition according to any one of claims 1 to 14, which is a dental resin cement.

**INTERNATIONAL SEARCH REPORT**

International application No.
**PCT/JP2022/037733**

**A. CLASSIFICATION OF SUBJECT MATTER**

***A61K 6/887***(2020.01)i; ***A61K 6/60***(2020.01)i; ***A61K 6/831***(2020.01)i; ***A61K 6/836***(2020.01)i; ***A61K 6/84***(2020.01)i
FI: A61K6/887; A61K6/831; A61K6/60; A61K6/836; A61K6/84

According to International Patent Classification (IPC) or to both national classification and IPC

**B. FIELDS SEARCHED**

Minimum documentation searched (classification system followed by classification symbols)

A61K6/887; A61K6/60; A61K6/831; A61K6/836; A61K6/84

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2008-88086 A (GC CORP.) 17 April 2008 (2008-04-17)<br>claims, paragraph [0038], examples | 1-15 |
| Y | JP 2009-144054 A (GC CORP.) 02 July 2009 (2009-07-02)<br>paragraph [0012], examples | 1-15 |
| Y | TSUKAGOSHI, Kou et al. Bond strength and computational analysis for silane coupling treatments on the adhesion of resin block for CAD/CAM crowns. Dental Mater. J. 2020, vol. 39 no. 5, pp. 844-854<br>title, abstract, beginning of the text, fig. 1, 10, 12, etc., tables 1, 3, etc. | 1-15 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 December 2022** | **27 December 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/037733**

| Patent document cited in search report | Publication date (day/month/year) | Patent family member(s) | Publication date (day/month/year) |
|---|---|---|---|
| JP 2008-88086 A | 17 April 2008 | (Family: none) | |
| JP 2009-144054 A | 02 July 2009 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019004391 A
- WO 2016007453 A
- WO 2017038218 A
- JP H0357916 B